# EUROPEAN PATENT SPECIFICATION

(11) **EP 0 998 451 B1**
(45) Date of publication and mention of the grant of the patent: **16.04.2003**
(21) Application number: 98920455.7
(22) Date of filing: 06.05.1998
(51) Int. Cl.: C07C 255/06, C07C 253/30, C07D 295/00, C07D 211/00, C07D 213/00, C07D 217/00, C07D 233/00, A61K 31/275, A61K 31/33

(54) **SUBSTITUTED 3,3-DIAMINO-2-PROPENENITRILES, THEIR PREPARATION AND USE**
SUBSTITUIERTE 3,3-DIAMINO-2-PROPENNITRILE, IHRE HERSTELLUNG UND VERWENDUNG
3,3-DIAMINO-2-PROPENENITRILES, PREPARATION ET UTILISATION DE CES DERNIERS

(30) Priority: 07.05.1997 DK 53397
(43) Date of publication of application: 10.05.2000
(73) Proprietor: NOVO NORDISK A/S, 2880 Bagsvaerd (DK)
(72) Inventor: DÖRWALD, Florenzio, Zaragoza, DK-2750 Ballerup (DK); HANSEN, John, Bondo, DK-4450 Jyderup (DK)
(74) Representative: Madsen, Inger Margrethe Schelde
(86) International application number: DK9800176
(87) International publication number: WO98050344

(56) References cited:
- EP-A- 0 010 396
- EP-A- 0 547 517
- EP-A- 0 591 891
- FR-A- 2 229 417
- US-A- 4 024 260
- US-A- 4 277 485
- STN INTERNATIONAL, File CA, CHEMICAL ABSTRACTS, Volume 73, No. 19, 9 November 1970, (Columbus, Ohio, US), Abstract No. 98434, "Disubstituted Acrylonitriles"; & JP,B,45 022 328 (TAKEDA CHEMICAL INDUSTRIES, LTD.) (28-07-70) Showa.
- CHEM. BER., Volume 114, 1981, KURT KLEMM et al., "3-Chlor-5-Dimethylamino-2-Formyl-4-Aza-2,4 -Pentadiennitril, Synthese und Umsetzungen mit Nucleophilen", pages 2001-2018.
- CHEM. REV., Volume 96, 1996, LORIN A. THOMPSON et al., "Synthesis and Applications of Small Molecule Libraries", pages 555-600.

## Description

### FIELD OF THE INVENTION

The present invention relates to substituted 3,3-diamino-2-propenenitriles, in the following also referred to as cyanoenamines, to methods for their preparation, to compositions comprising the compounds, to the use of these compounds as medicaments and their use in therapy e.g. in the treatment of diseases of the central nervous system, the cardiovascular system, the pulmonary system, the gastrointestinal system and the endocrinological system.

### BACKGROUND OF THE INVENTION

Potassium channels play an important role in membrane potential. Among the different types of potassium channels are the ATP-sensitive (K_{ATP}-) channels which are regulated by changes in the intracellular concentration of adenosine triphosphate. The K_{ATP}-channels have been found in cells from various tissues such as cardiac cells, pancreatic-cells, skeletal muscles, smooth muscles, central neurones and adenohypophysis cells. The channels have been associated with diverse cellular functions for example hormone secretion (insulin from pancreatic beta-cells, growth hormone and prolactin from adenohypophysis cells), vasodilation (in smooth muscle cells), cardiac action potential duration, neurotransmitter release in the central nervous system.

Modulators of the K_{ATP}-channels have been found to be of importance for the treatment of various diseases. Certain sulfonylureas which have been used for the treatment of non-insulin-dependent diabetes mellitus act by stimulating insulin release through an inhibition of the K_{ATP} -channels on pancreatic beta-cells.
The potassium channel openers, which comprise a heterogeneous group of compounds, have been found to be able to relax vascular smooth muscles and have therefore been used for the treatment of hypertension.

In addition, potassium channel openers can be used as bronchodilators in the treatment of asthma and various other diseases.

Furthermore, potassium channel openers have been shown to promote hair growth, and have been used for the treatment of baldness.

Potassium channel openers are also able to relax urinary bladder smooth muscle and therefore, can be used for the treatment of urinary incontinence. Potassium channel openers which relax smooth muscle of the uterus can be used for treatment of premature labour.

Since some K_{ATP}-openers are able to antagonize vasospasms in basilar or cerebral arteries the compounds of the present invention can be used for the treatment of vasospastic disorders such as subarachnoid haemorrhage and migraine.

Potassium channel openers hyperpolarizes neurons and inhibit neurotransmitter release and it is expected that the present compounds can be used for the treatment of various diseases of the central nervous system, e.g. epilepsia, ischemia and neurodegenerative diseases, and for the management of pain.

Recently, it has been shown that diazoxide (7-chloro-3-methyl-2H-1,2,4-benzothiadiazine 1,1-dioxide) and certain 3-(alkylamino)-4H-pyrido[4,3-e]-1,2,4-thiadiazine 1,1-dioxide derivatives inhibit insulin release by an activation of K_{ATP}-channels on pancreatic beta-cells (Pirotte B. et al. *Biochem. Pharmacol,* 47, 1381-1386 (1994); Pirotte B. et al., *J. Med. Chem.,* 36, 3211-3213 (1993). Diazoxide has furthermore been shown to delay the onset of diabetes in BB-rats ( Vlahos WD et al. *Metabolism* 40, 39-46 (1991). In obese zucker rats diazoxide has been shown to decrease insulin secretion and increase insulin receptor binding and consequently improve glucose tolerance and decrease weight gain (Alemzadeh R. et al. Endocrinol. 133, 705-712, 1993). It is expected that such potassium channel openers can be used for treatment of diseases characterised by an overproduction of insulin and for the treatment and prevention of diabetes.

### DESCRIPTION OF THE INVENTION

The present invention relates to substituted 3,3-diamino-2-propenenitriles, in the following also referred to as cyanoenamines, of the general formula I: 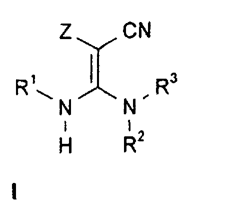 wherein
R¹ is alkyl optionally substituted with halogen, hydroxy, alkoxy, aryloxy, alkylthio, arylthio, dialkylamino, arylalkylamino or diarylamino; aralkyl, aryl optionally substituted with alkyl, trifluoromethyl, aryl, heteroaryl, halogen, alkoxy, aryloxy, dialkylamino, alkylarylamino, diarylamino, nitro, alkyl-sulfonyl, aryl-sulfonyl, cyano, alkoxycarbonyl or aminocarbonyl, heteroaryl optionally substituted with alkyl, aryl, heteroaryl, halogen, alkoxy, aryloxy, dialkylamino, alkylarylamino, diarylamino, halogen, nitro, alkyl-sulfonyl, aryl-sulfonyl, cyano, alkoxycarbonyl or aminocarbonyl;
R² and R³ are independently hydrogen, alkyl optionally substituted with aryl, heteroaryl, a 5-,6- or 7-membered heterocyclic system, halogen, hydroxy, alkoxy, aryloxy, alkylthio, arylthio, dialkylamino, arylalkylamino or diarylamino; aryl, optionally substituted with alkyl, aryl, heteroaryl, halogen, alkoxy, aryloxy, dialkylamino, alkylarylamino, diarylamino, nitro, alkyl-sulfonyl, aryl-sulfonyl, cyano, alkoxycarbonyl or aminocarbonyl; heteroaryl optionally substituted with alkyl, aryl, heteroaryl, halogen, alkoxy, aryloxy, dialkylamino, alkylarylamino, diarylamino, halogen, nitro, alkyl-sulfonyl, aryl-sulfonyl, cyano, alkoxycarbonyl or aminocarbonyl;
or R² and R³ are linked together by -(CH₂)ₙ-, n being 4-7,
provided that R² and R³ cannot be hydrogen at the same time;
Z is hydrogen; and
pharmaceutically acceptable salts thereof.

Within its scope the invention includes all diastereomers and enantiomers of compounds of formula I, some of which are optically active, and also their mixtures including racemic mixture thereof.

The scope of the invention also includes all tautomeric forms of the compounds of formula I.

The salts include pharmaceutically acceptable acid addition salts, pharmaceutically acceptable metal salts or optionally alkylated ammonium salts, such as hydrochloric, hydrobromic, hydroiodic, phosphoric, sulfuric, trifluoroacetic, trichloroacetic, oxalic, maleic, pyruvic, malonic, succinic, citric, tartaric, fumaric, mandelic, benzoic, cinnamic, methanesulfonic, ethane sulfonic, picric and the like, and include acids related to the pharmaceutically acceptable salts listed in *Journal of Pharmaceutical Science*, *66,* 2 (**1977**) and incorporated herein by reference, or lithium, sodium, potassium, magnesium and the like.

The term "5-,6- or 7-membered heterocyclic system" as used herein refers to: a monocyclic unsaturated or saturated system containing one, two or three hetero atoms selected from nitrogen, oxygen and sulfur and having 5 members, e.g. pyrrole, furan, thiophene, pyrroline, dihydrofuran, dihydrothiophene, imidazole, imidazoline, pyrazole, pyrazoline, oxazole, thiazole, isoxazole, isothiazole, 1,2,3-oxadiazole, furazan, 1,2,3-triazole, 1,2,3-thiadiazole or 2,1,3-thiadiazole; an aromatic monocyclic system containing two or more nitrogen atoms and having 6 members, e.g. pyrazine, pyrimidine, pyridazine, 1,2,4-triazine, 1,2,3-triazine or tetrazine; a non-aromatic monocyclic system containing one or more hetero atoms selected from nitrogen, oxygen and sulfur and having 6 or 7 members, e.g. pyran, thiopyran, piperidine, dioxane, oxazine, isoxazine, dithiane, oxathine, thiazine, piperazine, thiadiazine, dithiazine, oxadiazine or oxoazepane.

Alkyl refers to lower straight, cyclic, bicyclic, fused or branched alkyl having 1 to 15 carbon atoms, preferentially 1 to 6 carbon atoms. Aryl refers to phenyl or phenyl substituted with alkyl or phenyl, or phenyl fused with cycloalkyl, or polycyclic aromatic systems such as naphthyl, anthracenyl, phenanthrenyl, fluorenyl, etc. Alkylene refers to lower straight, cyclic, fused or branched alkylene having 1 to 15 carbon atoms, preferentially 1 to 6 carbon atoms. Heteroaryl refers to any of the possible isomeric, unsubstituted or alkyl-substituted pyrrolyl, furyl, thienyl, imidazolyl, pyrazolyl, triazolyl, tetrazolyl, oxazolyl, thiazolyl, oxadiazolyl, thiadiazolyl, pyridyl, pyrazinyl, pyrimidinyl and pyridazinyl, as well as the corresponding benzo and dibenzo derivatives or other fused ring-systems thereof. Heteroaryl is also intended to mean the partially or fully hydrogenated derivatives of the heterocyclic systems enumerated above. Alkoxy refers to -O-alkyl and aryloxy refers to -O-aryl. Cyano refers to -CN, hydroxy refers to -OH, amino refers to -NH₂ and nitro refers to -NO₂. Dialkylamino refers to -N(alkyl)₂. Alkylarylamino refers to -N(alkyl)(aryl) and diarylamino refers to -N(aryl)₂. Halogen refers to -F, -Cl, -Br and -I. Aralkyl refers to -alkylene-aryl. Alkylthio refers to -S-alkyl and arylthio refers to -S-aryl. Alkoxycarbonyl refers to -CO-O-alkyl and aminocarbonyl refers to -CO-N(alkyl)₂, -CO-N(alkyl)(aryl) or -CO-N(aryl)₂. Acylamino refers to -N(alkyl)-CO-alkyl or -N(alkyl)-CO-aryl. A leaving group refers to a group or atom capable of existing in solution as a negatively charged species, or a positively charged group or atom.

The compounds of the present invention interact with the potassium channels and hence act as openers or blockers of the ATP-regulated potassium channels, which make them useful in the treatment of various diseases of the cardiovascular system, e.g. cerebral ischemia, hypertension, ischemic heart diseases, angina pectoris and coronary heart diseases; the pulmonary system; the gastrointestinal system; the central nervous system and the endocrinological system.

The compounds of the present invention may also be used for the treatment of diseases associated with decreased skelettal muscle blood flow such as Reynauds disease and intermittent claudication.

Further, the compounds of the invention may be used for the treatment of chronic airway diseases, including asthma, and for treatment of detrusor muscle instability secondary to bladder outflow obstruction and therefore for kidney stones by aiding their passage along the ureter. Potassium channel openers also relax urinary bladder smooth muscle, thus, the compounds of the present invention can be used for the treatment of urinary incontinence.

The present compounds could also be used for treatment of conditions associated with disturbances in gastrointestinal mobility such as irritable bowel syndrome. Additionally these compounds can be used for the treatment of premature labor and dysmenorrhea.

Further, potassium channel openers promote hairgrowth, therefore, the compounds of the present invention can be used for the treatment of baldness.

In diseases such as nesidioblastosis and insulinoma in which a hypersecretion of insulin causes severe hypoglycemia the compounds of the present invention can be used to reduce insulin secretion. In obesity hyperinsulinemia and insulin resistance is very frequently encountered. This condition could lead to the development of noninsulin dependent diabetes (NIDDM). It is expected that potassium channel openers and hence the compounds of the present invention can be used for counteracting the hyperinsulinemia and thereby prevent diabetes and reduce obesity. In overt NIDDM treatment of hyperinsulinemia with potassium channel openers, and hence the present compounds, can be of benefit in restoring glucose sensitivity and normal insulin secretions.

In early cases of insulin dependent diabetes (IDDM) or in prediabetic cases, potassium channel openers and hence the present compounds can be used to induce betacell rest which may prevent the progression of the autoimmune disease.

Compounds of the present invention which act as blockers of K_{ATP} -channels can be used for the treatment of NIDDM.

Preferably, the compounds of the present invention may be used for treatment or prevention of diseases of the endocrinological system such as hyperinsulinaemia and diabetes.

Accordingly, in another aspect the invention relates to a compound of the general formula I or a pharmaceutically acceptable acid addition salt thereof for use as a therapeutically acceptable substance, preferably for use as a therapeutically acceptable substance in the treatment of hyperinsulinaemia and treatment or prevention of diabetes.

Furthermore, the invention also relates to the use of the inventive compounds of formula I as medicaments useful for treating hyperinsulinaemia and treating or preventing diabetes .

In yet another aspect, the present invention relates to methods of preparing the above mentioned compounds. The methods comprises solid phase and combinatorial synthesis of organic compounds, and most particularly, a therapeutically important class of compounds, namely differently substituted cyanoenamines, useful as potassium channel openers.
The new synthetic sequence disclosed in this invention gives access to a new class of cyanoenamines, useful as potassium channel openers.

The following terms are intended to have the following, general meanings:
1. Substrate: refers to any insoluble or partially insoluble material, to which compounds may be covalently attached. Substrates may be selected from the group consisting of any kind of organic or inorganic polymeric or oligomeric compound, e.g. polystyrene with different grades of crosslinking, polyethylene glycol (PEG), polyethylene glycol attached to polystyrene (e.g. TentaGel), polyacrylamides, polyamides, polysaccharides or silicates. Optionally, a given portion of substrate may be attached to a tag, i.e. a material or device which permits the unambiguos identification of this portion of substrate within a plurality of portions of substrate.
2. Linker: a molecule with at least two reactive sites, which permit its covalent attachment to other molecules or to a substrate. Either the bond of the linker to the substrate or the bond of the linker to other molecules attached to it or the linker itself must be cleavable upon selective exposure to an activator such as a selected chemical activator or other specific conditions, e.g. by treatment with a strong acid or by exposure to electromagnetic radiation or by metal catalysis.
3. Array: A collection of N single compounds or N mixtures of compounds with a common structural element, synthesized simultaneously in a parallel fashion using the same synthetic reaction sequence. The precise structure of a single compound within an array of compounds or the components of a mixture within an array of mixtures is determined by the sequence of reactants which gave rise to this compound or mixture and can be deduced from the recorded reaction-protocol. The spatial arrangement of the array is irrelevant.
4. Cyanoenamine: Organic compound containing the structural element RR'N-CR"=CR"'-CN.
5. Protecting group: A material which is chemically bound to a molecule or a substrate and which may be removed upon selective exposure to an activator such as a selected chemical activator or other specific conditions, e.g. by treatment with a strong acid or by exposure to electromagnetic radiation or by metal catalysis.
6. Combinatorial synthesis: An ordered strategy for parallel synthesis of arrays of single compounds or mixtures, by sequential addition of reagents.
7. Abbreviations: The following frequently used abbreviations are intended to have the following meanings:
   AcOH: glacial acetic acid
   DBU: 1,8-diazabicyclo[5.4.0]undec-7-ene
   DCM: dichloromethane, methylenechloride
   DIC: diisopropylcarbodiimide
   DMF: N,N-dimethyl formamide
   EDC: N-ethyl-N'-(3-dimethylaminopropyl)carbodiimide hydrochloride, "water-soluble carbodiimide"
   FMoc: fluorenylmethyloxycarbonyl
   NMP: N-Methylpyrrolidone
   R: organic radical
   TFA: trifluoroacetic acid
   THF: tetrahydrofuran

The method for the synthesis of therapeutically useful compounds is also provided by virtue of the present invention. The invention also provides a rapid approach for combinatorial synthesis and screening of arrays of cyanoenamine derivatives as a therapeutically important class of compounds. The invention also provides a solid phase synthesis of cyanoenamines, which eliminates purification and isolation steps and thus highly increases synthesis efficiency. The specification also describes an important extension of solid phase synthesis methods to nonoligomeric organic compounds.
A further understanding of the nature and advantages of the invention may be realized by reference to the remaining portions of the specification.

The application of the present invention also includes the rapid preparation and screening, preferably in parallel and simultaneous fashion, of a large number of differently substituted cyanoenamines of the general formula **I** 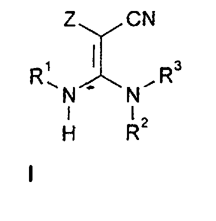 wherein
Z, R¹, R²and R³ are as defined above
or of the general formula **II** 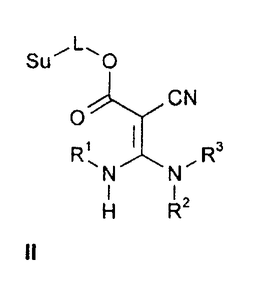 wherein
Su is a substrate,
L is a chemical bond or a linker,
R¹, R²and R³ are as defined above.

An overall illustration of the solid phase synthesis of cyanoenamines is shown in reaction Scheme 1.

### Scheme 1

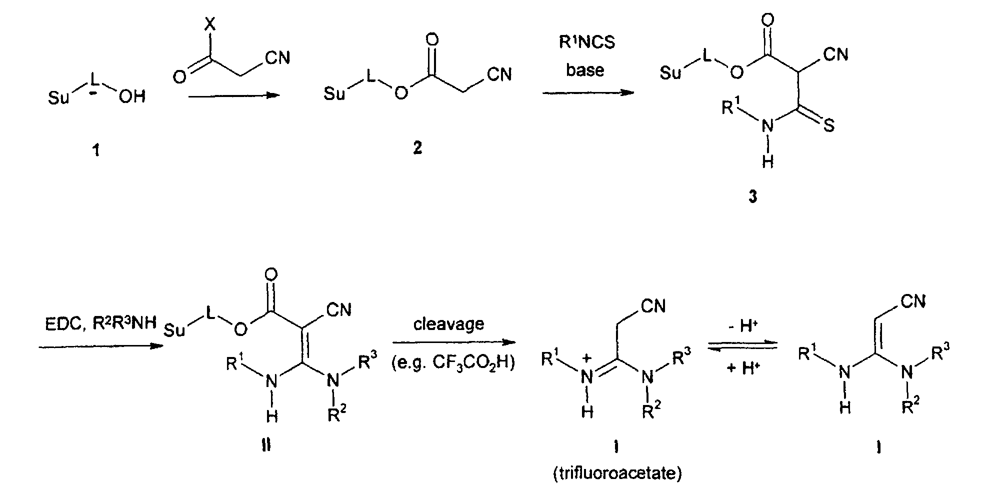

In this synthesis, the substrate Su may be any insoluble or partially insoluble material, to which compounds may be covalently attached. Preferentially, the substrates may be selected from the group consisting of polystyrene, polyethylene glycol (PEG), polyethylene glycol attached to polystyrene (e.g. TentaGel), polyamides, polysaccharides and silicates. Depending on the type of substrate chosen, different types of solvents or protecting groups may be used.
The substrate-bound alcohol **1** may be acylated with an appropriate cyanoacetic acid derivative of the general structure NC-CH₂-COX, X being a leaving group, preferentially with the *in situ* generated symmetric anhydride (Zaragoza, F. *Tetrahedron Lett.* **1995**, *36,* 8677-8678). Alternatively, other, *in situ* generated or isolated derivatives of cyanoacetic acid may be used as acylating reagents, such as the mixed anhydrides derived from alkyl chloroformates and cyanoacetic acid, or the imidazolide or other types of activated esters, such as the N-hydroxybenzotriazolyl ester or N-hydroxysuccinyl ester or other activated esters, obvious to those skilled in the art. The esterification reaction can optionally be carried out in the presence of a catalyst, e.g. 4-dimethylaminopyridine, to yield a derivative of the general formula {substrate}-{linker}-O-CO-CH₂-CN.
The resulting, resin bound cyanoacetic acid derivative 2 may then be treated with an excess of an aromatic or aliphatic isothiocyanate of the general structure R¹-NCS in an appropriate solvent such as NMP, DMF or THF, in the presence of a base, preferentially diisopropylethylamine or 1,8-diazabicyclo[5.4.0]undec-7-ene (DBU).

The resulting intermediate product **3** may then be treated with EDC and a primary or secondary aliphatic or aromatic amine of the general structure R²R³NH in a suitable solvent, such as NMP, DMF, acetonitrile, DCM, 1,2-dichloroethane, toluene, ethyl acetate, etc., preferentially in DMF. Alternatively, other condensing agents may be used (e.g. benzotriazole-1-yl-oxy-tris-(dimethylamino)phosphonium hexafluorophosphate ("BOP"), carbonyldiimidazole, N-ethyl-N'-(3-trimethylammoniumpropyl)-carbodiimide, diisopropylcarbodiimide, dicyclohexylcarbodiimide, etc.), alone or in the presence of a catalyst such as pyridinium tosylate or salts of *tert*iary amines. This reaction is closely related to a published procedure for the conversion of thioureas to cyanoguanidines (K. S. Atwal, S. Z. Ahmed, B. C. O'Reilly, *Tetrahedron Lett*. **1989**, *30*, 7313-7316).
Cleaving of the linker of the substrate-bound cyanoenamine **II** may release the cyanoenamine derivative **I** into solution. Cleavage conditions will depend upon the type of substrate and linker chosen. E. g., in the case of a polystyrene resin with a Wang linker or a Rink linker, treatment of the support-bound cyanoenamine **II** with neat TFA or TFA/DCM mixtures may lead to a cleavage of the linker. Thereby the cyanoenamine **I** may result as its cyanoamidine-trifluoroacetate tautomer, which may tautomerize reversibly into the neutral cyanoenamine when treated with bases or a buffer.
Alternatively, further chemical transformations may be carried out with the cyanoenamine derivatives **II**. Those cyanoenamines **II** which contain a NH-group may be acylated at nitrogen by treatment with an excess of an activated carboxylic acid derivative or with an isocyanate or with an isothiocyanate or with a sulfonylchloride, to yield the corresponding carboxamides, ureas, thioureas or sulfonamides, respectively. Each of these reactions may be performed by conventional means, readily apparent to those skilled in the art.
Alternatively, further chemical transformations may be carried out with the cyanoenamine derivatives I, which give high yields and pure crude products, so that no further purification of these derivatives will be required for their screening. For instance, those cyanoenamine derivatives which contain a NH-group may be acylated at nitrogen by treatment with an excess of an activated carboxylic acid derivative or with an isocyanate or with an isothiocyanate or with a sulfonylchloride, to yield the corresponding carboxamides, ureas, thioureas or sulfonamides, respectively. Each of these reactions may be performed by conventional means, readily apparent to those skilled in the art.
Using this synthetic method, arrays of cyanoenamine derivatives **II** or **I** may be constructed with the help of a device for parallel solid phase synthesis. This may be either the pin method developed by Geysen et al. (*J. Immunol. Meth.* **1987**, *102*, 259-274) or a device with several reactors for solid phase synthesis (containers with a permeable wall), which permits the automated or manual addition of reagents and solvents, as well as the removal of the solvents from the reactors by simultaneous or individual application of a pressure difference between the inside and the outside of the permeable wall of the reactors.
Such an array may be prepared on a multiple organic synthesizer (e.g. "ACT 496" of "Advanced ChemTech") by individually reacting under the conditions specified below a cyanoacetylated substrate located in individual containers, with different isothiocyanates of the general structure R¹-NCS in the presence of a base. The resulting intermediates **3** may then be reacted with different amines of the general structure R²R³NH in DMF in the presence of EDC to give an array of different cyanoenamine derivatives **II**. Cleavage from the support will provide an array of the corresponding cyanoenamines **I**.
The present invention also provides the synthesis of arrays of mixtures of cyanoenamine derivatives. This can be achieved either by the "split and mix" method (Sepetov, N.F., Krchnák, V., Stankova, M., Wade, S., Lam, K.S., and Lebl *Proc. Natl. Acad. Sci. USA* **1995**, *92*, 5426-5430) or by using mixtures of the corresponding reagents.
By virtue of the present invention basically two different types of arrays of cyanoenamines **I** or **II** may be constructed: fully combinatorial arrays (FCA) and not-fully combinatorial arrays (NFCA).
By FCA we refer to arrays of substituted cyanoenamines, in which all the possible combinations of a set of selected building blocks (R-groups) are realized. As an example, a FCA of N cyanoenamines may be prepared by selecting n isothiocyanates and m amines so that n x m = N, and synthesizing all the possible combinations of isothiocyanate/amine. The selection of building blocks may be done with regard to the expected properties of the members of the array.
By NFCA we refer to arrays of cyanoenamines, in which only a selection of all the possible combinations of a set of selected building blocks is realized. As an example, a NFCA of N cyanoenamines may be prepared by first selecting n isothiocyanates and m amines so that n x m > N. Then a selection of N cyanoenamines from all the n x m theoretically possible cyanoenamines is done by grouping all the n x m possible cyanoenamines into N groups of cyanoenamines with similar expected properties and selecting from each of these groups one cyanoenamine, which is then synthesized. The selection of building blocks and of cyanoenamines may be done with regard to the expected properties of the members of the array. For the preparation of such arrays of compounds, the exact positions of the substrate does, by itself, not give any structural information about the compound prepared on this particular batch of substrate. For this reason, the spatial arrangement of the substrate is irrelevant. Structural information will be accessible from the records of the sequences of reagents added to each batch of substrate. In every step of the preparation of a FCA or a NFCA, the exact location of one substrate-container within the array of containers and the structure of the different reagents added to this container is recorded, so that the precise structure of the cyanoenamine resulting from one given container can always be deduced.
The resulting arrays of cyanoenamines may then be screened by comparing the individual cyanoenamines in terms of their ability to bind to a particular receptor or to induce a particular biological process or to catalyze a biochemical or chemical reaction. This can be achieved basically in two different ways. One possibility may be the screening of the substrate-bound cyanoenamines **II**, e.g. against a soluble receptor. This could for instance be a radioactively labelled peptide or enzyme, which would easily permit to determine the binding-strength of a given substrate-bound cyanoenamine **II** to this peptide by washing away the excess of radioligand used and determining the remaining radioactivity of each substrate-bound cyanoenamine **II**-peptide complex. Alternatively, as a further example, catalytic activity of the different substrate-bound cyanoenamines **II** for a given biological process or a chemical reaction may be measured by comparing the speed at which this biological process or a chemical reaction takes place in the presence and in the absence of a given substrate-bound cyanoenamine **I**I.
The second option for screening may consist in screening the cyanoenamines **I**, after having cleaved the linker of the substrate-bound cyanoenamines **II** and using appropriately charged and indexed Microtiter plates of similar multiwell arrangements, in solution against an optionally substrate-bound receptor or enzyme. The screening of soluble small molecules is conventional and well known. Typically, radioassays are being used, in which the competitive binding of the radiolabelled, natural ligand of a given receptor and the compound to be tested for binding to this receptor is investigated.
As an example the cyanoenamines may be screened for the potassium channel opening a-citivity. This can be achieved by first treating rat aorta with ⁸⁶Rb⁺, and then with the cyanoenamines **I**. The ratio of radioactive ⁸⁶Rb⁺ released into the solution and the radioactivity remaining in the tissue may be proportional to the potassium channel opening activity of the tested cyanoenamine **I**. This type of essay has been described in literature (see e.g. T. Nakajima, T. Izawa, T. Kashiwabara, S. Nakajima, Y. Munezuka, *Chem. Pharm. Bull.* **1994**, *42*, 2475-2490).

Cyanoenamines as **I** can also be prepared in solution. The method used is sketched below:

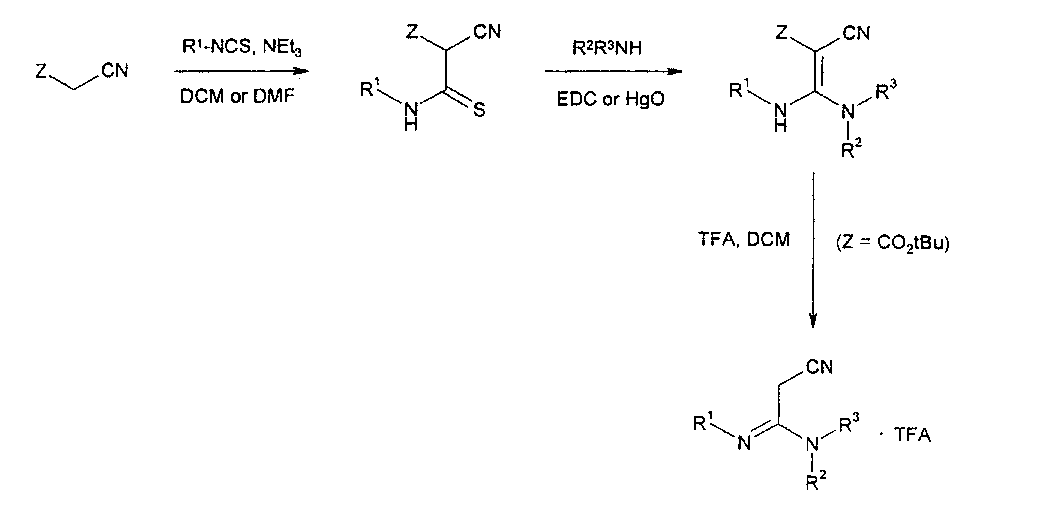

Acceptor substituted acetonitriles were reacted with isothiocyanates in the presence of a base. The resulting thioamides were treated with primary or secondary amines in the presence of a desulfurizing agent, as for instance mercury(II) oxide or EDC, to give cyanoenamines as **I**. Unsubstituted cyanoacetamidines could be prepared from cyanoenamines **I** with Z = *tert*-butyloxycarbonyl by treatment with trifluoroacetic acid in dichloromethane. Thereby hydrolysis of the estergroup, followed by decarboxylation occurs, to yield the corresponding cyanoacetamidine trifluoroacetates.

### PRIOR ART

Some derivatives of 2-cyano-3-(dimethylamino)-3-arylamino-2-propenenitriles have been claimed to be angiotensin II antagonists (EP 591891, *Chem. Abstr.* **1995**, *122*, 81364; *Chem. Abstr.* **1994,** *121*, 300890). Example:

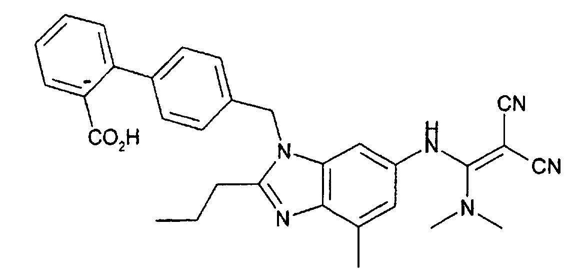

Other compounds containing this substructural element have been claimed to be antithrombotics (EP 547517, *Chem. Abstr.* **1993**, *119*, 249845; *Chem. Abstr.* **1993,** *119*, 180666), e.g.:

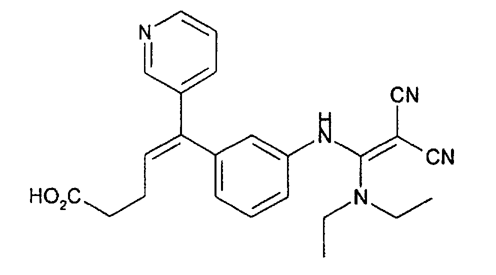

Several 3-(arylamino)-3-(alkylamino)-2-cyano-2-propenenitriles and -2-acrylamides have been claimed as fungicides and herbicides (EP 10396, *Chem. Abstr.* **1982,** *97*, 140276; *Chem. Abstr.* **1980**, *93*, 144701), some examples being:

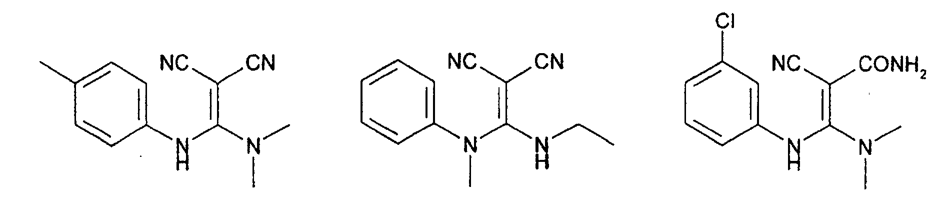

The reaction of amines RR'NH with mono-imidates of malononitrile of the general formula NC-CH₂-C(OR)=NH give compounds of the type RR'N-C(NH₂)=CH-CN, where one of the two amino groups is limited to be NH₂ (Cocco, M. T.; Congiu, C.; Maccioni, A.; Plumitallo, A., *J. Heterocycl. Chem.,* **1989**, *26*, 1859-1862; Klemm, K.; Pruesse, W.; Baron, L.; Daltrozzo, E., *Chem. Ber*., **1981**, *114*, 2001-2018; Cocco, M. T.; Onnis, V., *Synthesis,* **1993**, *2*, 199-201; Fanshawe, W. J. et al., *J. Org. Chem.,* **1964**, *29*, 308-311; Troschuetz, R.; Dennstedt, T., *Arch. Pharm. (Weinheim Ger.)*, **1994**, *327*, 85-90).
A further method consists in the reaction of O-alkylated cyanoacetamides with aliphatic amines (G. J. Durant et al., patent, CH 606026, *Chem. Abstr.* **1979**, *90*, 87449, G. J. Durant, patent, US 4024260, *Chem. Abstr*., **1977**, *87*, 135327). Also the reaction of 3,3-dimethoxyacrylonitrile with amines, which can be carried out stepwise in order to prepare compounds of the general formula RR'N-C(NR"R"')=CH-CN, has been reported (G. J. Durant, patent, US 4277485, *Chem. Abstr*., **1981**, *95*, 156591) and used for the preparation of ranitidine-analogues.
Moreover, the reaction of 3,3-dichloroacrylonitrile with amines has been reported to give cyanoenamines of the general structure (RR'N)₂C=CH-CN, with two identical amine-moieties RR'N- (Hashimoto et al., *J. Org. Chem.,* **1970,** *35*, 828-831; Takeda Chem.Ind.Ltd., JP 7022328, **1970**, Chem.Abstr., **73**, 98434z). In addition to these, some special methods for the synthesis of these compounds have been described (e.g. Sasaki, T.; Kojima, A. *J. Chem. Soc. Sec. C,* **1970**, 476-480; Clark, J., Parvizi, B., Southon, I. W., *J. Chem. Soc*., *Perkin Trans. l*, **1976**, 125-130; Smith; Kline and French Lab. Lim, FR 2229417, DE 2423813, Chem. Abstr., **82**, 170943; Meyer; K., *Justus Liebigs Ann. Chem.,* **1978**, 1491; Elagamey, A. G. A.; El-Taweel, F. M. A., *J. Prakt. Chem.,* **1991**, *333*, 333-338).
For the preparation of 2-acceptor-substituted 3,3-bis(alkyllarylamino)-2-propenenitriles, several different synthetic methods have been described (Elvidge, J. A. et al., *J. Chem. Soc., Perkin Trans. 1*, **1983**, 1741-1744; Yatsishin, A. A.. et al., *Zh. Org. Khim.* **1979**, *15,* 1381-1384; Hartke, K., *Angew. Chem.* **1964**, *76*, 781)

### References

1. Gallop, M. A.; Barrett, R. W.; Dower, W. J.; Fodor, S. P. A.; Gordon, E. M. *J. Med. Chem.* **1994**, *37*, 1233-1251.
2. Gordon, E. M.; Barrett, R. W.; Dower, W. J.; Fodor, S. P. A.; Gallop, M. A. *J. Med. Chem.* **1994**, *37*, 1385-1401.
3. Terrett, N. K.; Gardner, M.; Gordon, D. W.; Kobylecki, R. J.; Steele, J. *Tetrahedron.* **1995**, *51*, 8135-8173.
4. Lebl, M.; Krchnák, V.; Sepetov, N. F.; Kocis, P.; Patek, M.; Flegelova, Z.; Ferguson, R.; Lam, K. S. *Journal Of Protein Chemistry.* **1994**, *13*, 484-486.
5. Sepetov, N. F.; Krchnák, V.; Stankova, M.; Wade, S.; Lam, K. S.; Lebl, M. *Proc. Natl. A-cad. Sci. USA* **1995**, *92*, 5426-5430.
6. Liskamp, R. M. J. *Angew. Chem. Int. Ed. Engl.* **1994**, *33*, 633-636.
7. Houghten, R. A.; Kay, B. K.; Madden, D.; Krchnák, V.; Lebl, M.; Chabala, J. C.; Kauffman, S. *Perspectives in Drug Discovery and Design* **1994**, *2,* 249-325.
8. Seligmann, B.; Abdul-Latif, F.; Al-Obeidi, F.; Flegelova, Z. *European Journal Of Medicinal Chemistry* **1995**, *30*, 319-335.
9. Baldwin, J. J.; Burbaum, J. J.; Henderson, I.; Ohlmeyer, M. H. J. *J. Am. Chem. Soc.* **1995**, *117*, 5588-5589.
10. Jung et al., "Multiple Peptide Synthesis Methods and their Applications", *Angew. Chem. Int. Ed. Engl*. **1992**, *31*, 367-383.
11. J. A. Ellman, Solid phase and combinatorial synthesis of benzodiazepine compounds on a solid support, US Patent 5,288,514; Feb. 22, 1994.

### PHARMACOLOGICAL METHODS

The ability of the compounds to interact with potassium channels can be determined by various methods. When patch-clamp techniques (Hamill O.P., Marty A., Nefer E., Sakman B. and Sigworth F.J., *Plügers Arch.* **1981**, *391*, 85-100) are used the ionic current through a single channel of a cell can be recorded.

The activity of the compounds as potassium channel openers can also be measured as relaxation of rat aortas rings according to the following procedure:

A section of rat thoracic aorta between the aortic arch and the diaphragm was dissected out and mounted as ring preparations as described by Taylor P.D. et al., *Brit. J. Pharmacol.,* **1994,** *111*, 42-48.

After a 45 min. equilibration period under a tension of 2 g, the preparations were contracted to achieve 80% of the maximum response using the required concentration of phenylephrine. When the phenylephrine response reached a plateau, potential vasodilatory agents were added cumulatively to the bath in small volumes using half log molar increments at 2 min intervals. Relaxation was expressed at the percentage of the contracted tension. The potency of a compound was expressed as the concentration required to evoke a 50% relaxation of the tissue.

In the pancreatic b-cell the opening of the K_{ATP}-channels can be determined by measuring the subsequent change in the concentration of cytoplasmic free Ca²⁺ concentration according to the method of Arkhammer P. et al. , *J. Biol. Chem.* **1987, 262,** 5448-5454.

### ⁸⁶Rb⁺ efflux from a β-cell line

The RIN 5F cell line was grown in RPMI 1640 with Glutamax I, supplemented with 10% fetal calf serum (from GibcoBRL, Scotland, UK) and maintained in an atmosphere of 5% CO₂/95% air at 37 °C. The cells were detached with a Trypsin-EDTA solution (from GibcoBRL, Scotland, UK), resuspended in medium, added 1 mCi/mL ⁸⁶Rb⁺ and replated into microtiter plates (96 well cluster 3596, sterile, from Costar Corporation, MA, USA) at a density of 50000 cells/well in 100 µl/well, and grown 24 hours before use in assay.

The plates were washed 4 times with Ringer buffer (150 mM NaCI, 10 mM Hepes, 3.0 mM KCl, 1.0 mM CaCl₂, 20 mM sucrose, pH 7.1). Eighty µL Ringer buffer and 1 µL control- or test compound dissolved in DMSO was added. After incubation 1 h at room temperature with a lid, 50 µL of the supernatant was transferred to PicoPlates (Packard Instrument Company, CT, USA) and 100 µL MicroScint40 (Packard Instrument Company, CT, USA) added. The plates were counted in TopCount (Packard Instrument Company, CT, USA) for 1 min/well at the ³²P program.

The calculation of EC₅₀ and Eₘₐₓ was done by SlideWrite (Advanced Graphics Software, Inc., CA, USA) using a four parameter logistic curve: y = (a-d)/(1+(x/c)^{b})+d, where a = the activity estimated at concentration zero, b = a slope factor, c = the concentration at the middle of the curve and, d = the activity estimated at infinite concentration. EC₅₀ = c and Eₘₐₓ= d, when the curve is turned of at infinite concentrations.

The compounds according to the invention are effective over a wide dosage range. In general satisfactory results are obtained with dosages from about 0.05 mg to about 1000 mg, preferably from about 0.1 mg to about 500 mg, per day. A most preferable dosage is about 5 mg to about 200 mg per day. The exact dosage will depend upon the mode of administration, form in which administered, the subject to be treated and the body weight of the subject to be treated, and the preference and experience of the physician or veterinarian in charge.

The route of administration may be any route, which effectively transports the active compound to the appropriate or desired site of action, such as oral or parenteral e.g. rectal, transdermal, subcutaneous, intravenous, intramuscular or intranasal, the oral route being preferred.

Typical compositions include a compound of formula I or a pharmaceutically acceptable acid addition salt thereof, associated with a pharmaceutically acceptable excipient which may be a carrier or a diluent or be diluted by a carrier, or enclosed within a carrier which can be in form of a capsule, sachet, paper or other container. In making the compositions, conventional techniques for the preparation of pharmaceutical compositions may be used. For example, the active compound will usually be mixed with a carrier, or diluted by a carrier, or enclosed within a carrier which may be in the form of a ampoule, capsule, sachet, paper, or other container. When the carrier serves as a diluent, it may be solid, semi-solid, or liquid material which acts as a vehicle, excipient, or medium for the active compound. The active compound can be adsorbed on a granular solid container for example in a sachet. Some examples of suitable carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose and polyvinylpyrrolidone. The formulations may also include wetting agents, emulsifying and suspending agents, preserving agents, sweetening agents or flavouring agents. The formulations of the invention may be formulated so as to provide quick, sustained, or delayed release of the active ingredient after administration to the patient by employing procedures well known in the art.

The pharmaceutical preparations can be sterilized and mixed, if desired, with auxiliary agents, emulsifiers, salt for influencing osmotic pressure, buffers and/or colouring substances and the like, which do not deleteriously react with the active compounds.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, corn starch, and/or potato starch. A syrup or elixir can be used in cases where a sweetened vehicle can be employed.

A typical tablet, appropriate for use in this method, may be prepared by conventional tabletting techniques and contains:

| | |
|---|---|
| Active compound | 5.0 mg |
| Lactosum | 67.8 mg Ph.Eur. |
| Avicel® | 31.4 mg |
| Amberlite® | 1.0 mg |
| Magnesii stearas | 0.25 mg Ph.Eur. |

Due to their high degree of activity, the compounds of the invention may be administered to a mammal, especially a human, in need of such treatment, prevention, elimination, alleviation or amelioration of various diseases as mentioned above and especially of diseases of the endocrinological system such as hyperinsulinaemia and diabetes. Such mammals include also animals, both domestic animals, e.g. household pets, and non-domestic animals such as wildlife.

### Examples

### Example 1. Synthesis of N-(4-methoxybenzyl)-N'-phenylcyanoacetamidine trifluoroacetate

To a suspension of Wang resin (20.0 g, 19.2 mmol, Novabiochem, loading: 0.96 mmol/g) in DCM (100 mL) cyanoacetic acid (30.0 g, 353 mmol) and DMF (100 mL) were added. While stirring DIC (25 mL) was portionwise added, whereby an exothermic reaction took place. When the addition of DIC was completed, 4-dimethylaminopyridine (10 mL of a 1M solution in DMF) was added, and the resulting mixture stirred at room temperature for 15 h. The mixture was then filtered and the resin was washed extensively with DMF, DCM and methanol. After drying, approx. 20 g of Wang resin-O-CO-CH₂-CN was obtained.

To this resin (0.30 g, approx. 0.3 mmol, swollen in DCM) DMF (4 mL), diisopropylethylamine (0.8 mL) and phenylisothiocyanate (0.54 mL, 4.5 mmol) were added. The resulting mixture was shaken for 16 h, filtered, washed with DMF (3 x 6 mL) and a mixture of EDC (0.95 g, 4.95 mmol), DMF (5 mL) and 4-methoxybenzylamine (0.40 mL, 3.03 mmol) was then added. The mixture was shaken for 24 h, filtered, and the resin was carefully washed with DMF, methanol, DCM and 10% AcOH in DCM. It was then suspended in DCM (3 mL) and TFA (2 mL) and shaken for 35 min. Tetrachlorocarbon (5 mL) was the added, and after filtration and washing with DCM the filtrates were concentrated. Thereby 84 mg (71%) of N-(4-methoxybenzyl)-N'-phenylcyanoacetamidine trifluoroacetate were obtained as an oil, which slowly crystallized at room temperature within 48 h. Recrystallization (ethyl acetate/methanol/heptane) yielded 22 mg of the title compound as colourless crystals, mp 161-163°C.

LCMS (Lichrosorb RP 18, acetonitrile/water gradient, monitored at 214 nm): elution at 7.2 min; MH⁺ calcd.: 280, found: 280. ¹H NMR (300 MHz, CDCl₃/DMSO-*d*_{*6*} 1:1) δ 3.82 (s, br, 3H), 3.89 (s, br, 2H, exchangeable with D₂O), 4.60 (s, br, 2H), 6.95 (d, *J* = 8.0 Hz, 2H), 7.29 (d, *J* = 8.0 Hz, 2H), 7.30-7.55 (m, 5H), 10.25 (s, br, 1H); ¹³C NMR (75 MHz, DMSO-*d*_{*6*}) δ 20.26 (t), 46.17 (t), 55.10 (q), 113.98 (d), 125.66 (d), 126.71 (d), 127.62 (d), 129.19 (d), 137.50 (s, br), 154.36 (s, br), 159.29 (s). Anal. Calcd. for C₁₉H₁₈F₃N₃O₃ (393.36): C, 58.01; H, 4.61; N, 10.68. Found: C, 57.98; H, 4.68; N, 10.47.

### Example 2. Synthesis of 2-cyano-3-(3-methylbutylamino)-3-(phenylamino)-2-propenenitrile

To a solution of malononitrile (0.34 g, 5.15 mmol) in DMF (5 mL) at 0 °C first phenylisothiocyanate (0.60 mL, 5.02 mmol) and then triethylamine (1.4 mL) were added. The resulting mixture was stirred at 0 °C for 25 min, and then a freshly prepared mixture of EDC (2.90 g, 15.1 mmol), 3-methylbutylamine (1.20 mL, 10.3 mmol) and DMF (10 mL) was added. After stirring at room temperature for 2 d the mixture was poured into a mixture of ice-water (50 mL) and conc. HCI (3.0 mL). The product was extracted (2 x 30 mL ethyl acetate), the combined organic extracts were washed (2 x 30 mL brine), dried (magnesium sulfate) and concentrated. The remaining oil was mixed with methanol (3 mL), whereby a solid precipitated. After 20 h the solid was filtered off and dried in vacuo. 0.60 g (47%) of the title compound was obtained as slightly yellow crystals. Recrystallization from ethyl acetate/heptane yielded 0.40 g of an analytically pure sample, mp. 183-185 °C. LCMS: MH⁺ calcd.: 255, found: 255. ¹H NMR (400 MHz, DMSO-*d*_{*6*}): δ= 0.83 (d, *J* = 7 Hz, 6H), 1.40 (q, *J*= 7 Hz, 2H), 1.57 (nonett, *J* = 7 Hz, 1H), 3.18 (m, 2H), 7.10 (m, 3H), 7.33 (t, *J* = 8 Hz, 2H), 7.87 (s, br, 1H), 9.33 (s, 1H); ¹³C NMR (100 MHz, DMSO-*d*_{*6*}): δ = 22.06, 24.90, 35.34, 37.38, 41.92, 118.11, 121.46, 124.16, 129.15, 138.81, 161.43. Anal. Calcd. for C₁₅H₁₈N₄ (254.34): C, 70.84; H, 7.13; N, 22.03. Found: C, 70.88; H, 7.28; N, 21.90.

### Example 3. Synthesis of 3-[3,5-bis(trifluoromethyl)phenylamino]-2-cyano-3-(3-methylbutylamino)acrylic acid tert-butyl ester

To a solution of 2-{N-[3,5-bis(trifluoromethyl)phenyl]thiocarbamoyl}cyanoacetic acid *tert*-butylester (1.12 g, 2.7 mmol, prepared from cyanoacetic acid *tert*-butyl ester and 3,5-bis(trifluoromethyl)phenylisothiocyanate) in DCM (15 mL) 3-methylbutylamine (1.0 mL, 8.60 mmol), magnesium sulfate (0.5 g) and mercury(II) oxide (2.0 g) were added. The resulting mixture was stirred at room temperature for 14 h, diluted with DCM (30 mL), filtered over Celite, washed with ice-cold diluted hydrochloric acid, with brine (2 x 20 mL), dried (magnesium sulfate) and concentrated. The residue was crystallized from heptane. 0.29 g (23%) of the title compound was obtained as colourless crystals, mp. 128-129 °C. From the mother liquor additional 0.25 g (20%) of product were obtained. ¹H NMR (400 MHz, DMSO-*d*_{*6*}): δ= 0.82 (d, *J* = 7 Hz, 6H), 1.41 (s, 9H), 1.43 (q, *J* = 7 Hz, 2H), 1.57 (nonett, *J* = 7 Hz, 1H), 3.18 (q, br, *J* = 7 Hz, 2H), 7.63 (s, 2H), 7.70 (s, 1H), 8.91 (s, br, 1H), 9.52 (s, 1H); ¹³C NMR (100 MHz, DMSO-*d*_{*6*}): δ= 21.96, 24.95, 28.02, 37.50, 41.84, 61.55, 79.66, 115.53, 118.67, 119.87, 123.05 (q, *J* = 282 Hz), 130.44 (q, *J* = 33 Hz), 142,11, 160.46, 167.93. Anal. Calcd. for C₂₁H₂₅F₆N₃O₂ (465.44): C, 54.19; H, 5.41; N, 9.03. Found: C, 54.27; H, 5.57; N, 8.84.

### Example 4. Synthesis of 3-[3,5-bis(trifluoromethyl)phenylamino]-3-(3-methylbutylamino)-2-propenenitrile

To a solution of 3-[3,5-bis(trifluoromethyl)phenylamino]-2-cyano-3-(3-methylbutylamino)acrylic acid tert-butyl ester (194 mg, 0.417 mmol) in DCM (2 mL) trifluoroacetic acid (2 mL) was added. After 30 min at room temperature the solution was concentrated, the residue was redissolved in carbon tetrachloride (10 mL) and reconcentrated. The product was purified by flash chromatography (6 g silica gel, gradient of heptane/ethyl acetate). 45 mg (30%) of the title compound was obtained as an oil. LCMS: MH⁺ = 366. This compound was identical by LCMS to the product obtained from solid-phase synthesis,

### Example 5. Synthesis of 3-[3,5-bis(trifluoromethyl)phenylamino]-2-cyano-3-(3-methylbutylamino)-2-propenenitrile

To a solution of malononitrile (0.33 g, 5.00 mmol) in DCM at 0 °C first 3,5-bis(trifluoromethyl)phenylisothiocyanate (1.41 g, 5.20 mmol) and then triethylamine (1.0 mL) were added. The mixture was stirred at room temperature for 1 h 45 min and then DCM (5 mL), 3-methylbutylamine (1.0 mL, 8.60 mmol), magnesium sulfate (0.57 g) and mercury(II) oxide (2.54 g, 11.7 mmol) were added. The resulting mixture was stirred at room temperature for 17 h, whereby it turned black. This mixture was then filtrated (Celite), diluted with DCM (60 mL), washed with an ice-cold mixture of water (100 mL) and conc. HCI (2.0 mL), with brine (3 x 50 mL), dried (magnesium sulfate) and concentrated. The residue was purified by column chromatography (50 g silica gel, heptane/ethyl acetate gradient), to yield 1.22 g (63%) of the title compound as a foam. This foam could be crystallized from toluene/heptane, yielding 1.08 g of almost colourless crystals, mp. 158-159 °C. ¹H NMR (300 MHz, DMSO-*d*_{*6*}): δ= 0.87 (d, *J* = 7 Hz, 6H), 1.45 (q, *J* = 7 Hz, 2H), 1.60 (nonett, *J* = 7 Hz, 1H), 3.31 (m, 2H), 7.72 (s, 2H), 7.78 (s, 1H), 8.22 (s, br, 1H), 9.79 (s, 1H); ¹³C NMR (75 MHz, DMSO-*d*_{*6*}): δ = 22.04, 24.99, 36.85, 37.14, 59.66, 116.52, 117.52, 121.21, 123.02 (q, *J* = 275 Hz), 131.02 (q, *J* = 33 Hz), 141,35, 161.27. Anal. Calcd. for C₁₇H₁₆F₆N₄ (390.33): C, 52.31; H, 4.13; N, 14.35. Found: C, 52.80; H, 4.29; N, 13.83.

### Example 6. Synthesis of 3-[3,5-bis(trifluoromethyl)phenylamino]-3-(3-methylbutylamino)-2-(4-chlorophenylsulfonyl)-2-propenenitrile

To a solution of 3,5-bis(trifluoromethyl)phenylisothiocyanate (1,44 g, 5.31 mmol) in DCM (15 mL) and acetonitrile (5 mL) first 4-chlorophenylsulfonylacetonitrile (1.10 g, 5.10 mmol) and then triethylamine (1.0 mL) were added. The resulting mixture was stirred at room temperature for 2 h 15 min, and then isoamylamine (0.65 mL, 5.59 mmol), mercury(II) oxide (2.70 g, 12.47 mmol) and magnesium sulfate (0.8 g) were added. Stirring was continued for 2 d. The mixture was then filtered, poured into a mixture of ice-water (200 mL) and concentrated hydrochloric acid (2 mL), phases were separated, the aqueous layer was extracted twice with DCM (20 mL) and the combined extracts were dried (magnesium sulfate) and concentrated. Column chromatography of the residue (100 g silica gel, gradient elution with heptane/ethyl acetate 10:0 to 3:1) gave 317 mg (12%) of the title compound as an oil. ¹H NMR (300 MHz, DMSO-*d*_{*6*}): δ = 0.79 (d, *J* = 7 Hz, 6H), 1.39 (q, *J* = 7 Hz, 2H), 1.50 (nonett, *J* = 7 Hz, 1H), 3.31 (m, 2H), 7.27 (s, 2H), 7.67-7.80 (m, 5H), 8.13 (s, br, 1H), 9.76 (s, 1H); LCMS: elution at 16.23 min, MH⁺: 540.

### Example 7. Synthesis of 3-[3,5-bis(trifluoromethyl)phenylamino]-2-cyano-3-[3-(2-oxoazepan-1-yl)propylamino]acrylic acid tert-butyl ester

To a mixture of 3,5-bis(trifluoromethyl)phenylisothiocyanate (2.83 g, 10.4 mmol), DCM (20 mL) and *tert*-butyl cyanoacetate (1.65 mL, 11.6 mmol) DBU (4.0 mL, 26.8 mmol) was added, whereby an exothermic reaction occurred. The resulting mixture was stirred at room temperature for 2 h, and then magnesium sulfate (2.0 g), isoamylamine (2.35 mL) and mercury(II) oxide (6.80 g, 31.4 mmol) were added. After stirring for 25 h, the mixture was mixed with celite, filtered, and the filtrate was mixed with 0.5 M hydrochloric acid. After an additional filtration the filtrate was extracted (3 x 100 mL DCM), the combined extracts were washed with brine (2 x 300 mL, emulgates strongly), dried (magnesium sulfate) and concentrated. Column chromatography (80 g silica gel, gradient elution with heptane/ethyl acetate 10:0 to 2:3) gave 2.23 g (39%) of the title compound as a colourless solid, along with 0.72 g (15%) of 3-[3,5-bis(trifluoromethyl)phenylamino]-2-cyano-3-(3-methylbutylamino)acrylic acid *tert*-butyl ester. A sample of the title compound was further purified by recrystallization. Colourless solid, mp. 167-168 °C (ethyl acetate). LCMS: elution at 15.4 min, MH⁺: 549; ¹H NMR (400 MHz, DMSO-*d*_{*6*}): δ = 1.40 (s, 9H), 1.43 (m, 4H), 1.57 (m, 2H), 1.71 (q, *J* = 7 Hz, 2H), 2.35 (m, 2H), 3.20 (q, *J* = 7 Hz, 2H), 3.31 (m, 2H), 7.66 (s, 2H), 7.68 (s, 1H), 8.95 (s, br, 1H), 9.52 (s, 1H); ¹³C NMR (100 MHz, DMSO-*d*_{*6*}): δ = 22.93, 27.93, 28.11, 28.32, 29.23, 36.44, 41.55, 44.47, 48.38, 61.93, 79.66, 115.36, 118.75, 119.89, 123.18 (q, *J* = 275 Hz), 131.02 (q, *J* = 33 Hz), 142.33, 160.59, 167.95, 174.74. Anal. Calcd. for C₂₅H₃₀F₆N₄O₃ (548.53): C, 54.74; H, 5.51; N, 10.21. Found: C, 54.62; H, 5.66; N, 9.97.

### Example 8. Synthesis of 3-[3,5-bis(trifluoromethyl)phenylamino]-3-[3-(2-oxoazepan-1-yl)propylamino]acrylonitrile

To a solution of 3-[3,5-bis(trifluoromethyl)phenylamino]-2-cyano-3-[3-(2-oxoazepan-1-yl)propylamino]acrylic acid *tert*-butyl ester (203 mg, 0.37 mmol) in DCM (6.0 mL) at 0 °C TFA (6.0 mL) was added. The resulting mixture was stirred at 0 °C for 30 min and then poured into an ice-cold, aqueous, saturated NaHCO₃-solution (100 mL). After dilution with DCM (30 mL) phases were separated, the aqueous layer was extracted (3 x 20 mL DCM), the combined extracts were washed with brine (2 x 50 mL), dried (magnesium sulfate) and concentrated, to yield 190 mg (100%) of the title compound as an oil (mixture of isomers). LCMS: elution at 9.97 min, MH⁺: 449). ¹H NMR (300 MHz, DMSO-*d*_{*6*}): δ = 1.45-1.80 (m, 8H), 2.40 (m, 2H), 2.95 (m, 1H), 3.21 (m, 2H), 3.35 (m, 6H), 3.58 (s, br, 1H), 7.35-7.70 (m, 3H).

### Example 9. Synthesis of 2-cyano-3-(4-methoxybenzylamino)-3-(phenylamino)acrylic acid tert-butyt ester

To a solution of *tert*-butyl cyanoacetate (1.3 mL, 9.12 mmol) in DMF (10 mL) at 0 °C first phenylisothiocyanate (1.2 mL, 10.0 mmol) and then triethylamine (2.80 mL) were added. The resulting mixture was stirred at 0 °C for 30 min and at room temperature for 30 min. The mixture was then cooled down to 0 °C and EDC (5.77 g, 30.1 mmol), DMF (30 mL) and 4-methoxybenzylamine (2.65 mL, 20.3 mmol) were added. After stirring for 9 h at room temperature, for 14 h at 60 °C and then for 2d at room temperature, the mixture was poured on ice (150 ml) and conc. HCI (4 mL). Extraction (3 x 30 mL ethyl acetate), drying of the combined extracts (magnesium sulfate) and concentration yields an oil, which is purified by column chromatography (50 g silica gel, gradient elution with heptanelethyl acetate 1:0 to 1:4). 0.43 g (12%) of the title compound are obtained as a colourless solid, mp. 158-159 °C (ethyl acetate/heptane). ¹H NMR (400 MHz, DMSO-*d*_{*6*}): δ = 1.39 (s, 9H), 3.72 (s, 3H), 4.22 (s, br, 2H), 6.89 (d, *J* = 8 Hz, 2H), 7.02-7.16 (m, 5H), 7.32 (m, 2H), 8.95 (s, br, 0.5H), 9.18 (s, 0.5H). Anal. Calcd. for C₂₂H₂₅N₃O₃ (379.46): C, 69.64; H, 6.64; N, 11.07. Found: C, 69.51; H, 6.80; N, 10.89.

### Example 10. Automated synthesis of an array of eighty different cyanoenamines

An array of eighty different cyanoenamines has been prepared in the following way: Into eighty reactors of the multiple organic synthesizer "ACT 496" of "Advanced ChemTech" 100 mg of Wang-resin-bound cyanoacetic acid (prepared as described in example 1) was equally distributed. Then each of the eighty reactors was treated as described in example 1 with one of 8 different aromatic isothiocyanates, namely 4-trifluoromethylphenylisothiocyanate, 2-trifluoromethylphenylisothiocyanate, 2,3-dichlorophenylisothiocyanate, 3-chloro-4-fluorophenylisothiocyanate, 2-methoxy-4-nitrophenylisothiocyanate, 2,4-difluorophenylisothiocyanate, 4-cyanophenylisothiocyanate and 3,5-bis(trifluoromethyl)phenylisothiocyanate. The resulting thioamides were then treated with ten different primary amines, namely with 2-methylpropylamine, 1,2-dimethylpropylamine, isopropylamine, 1,3-dimethylbutylamine, 2,2-dimethylpropylamine, butylamine, 4-*tert*-butylcyclohexylamine, 1,2,2-trimethylpropylamine, exo-2-norbomylamine and cyclohexylmethylamine in such a way, that all possible combinations of isothiocyanateamine were realized. After extensive washing, the resulting resin-bound cyanoenamines were cleaved from the substrate by treatment with 50% TFA in DCM (30 min), yielding an array of eighty different cyanoenamines in purities of 70->90% (HPLC). The samples were redissolved several times in methanol and concentrated again in order to remove traces of TFA. Finally the samples were redissolved in methanol (2 mL) and triethylamine (0.05 mL), concentrated again and redissolved in DMSO (3.5 mL). The resulting solutions were used for the screening.

Following the procedure given above, the following cyanoenamine derivatives I have been prepared:

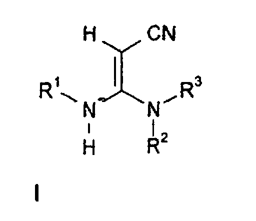

| | | | **MH**^{**+**} | |
|---|---|---|---|---|
| **No** | **R**^{**1**} | **-NR**^{**2**}**R**^{**3**} | **expctd** | **found** |
| 1 | 4-(trifluoromethyl)phenyl | 2-(methylpropyl)amino | | |
| 2 | 4-(trifluoromethyl)phenyl | (1,2-dimethylpropyl)amino | | |
| 3 | 4-(trifluoromethyl)phenyl | isopropylamino | | |
| 4 | 4-(trifluoromethyl)phenyl | (1,3-dimethylbutyl)amino | | |
| 5 | 4-(trifluoromethyl)phenyl | (2,2-dimethylpropyl)amino | | |
| 6 | 4-(trifluoromethyl)phenyl | butylamino | | |
| 7 | 4-(trifluoromethyl)phenyl | (4-*tert*-butylcyclohexyl)amino | 366 | 366 |
| 8 | 4-(trifluoromethyl)phenyl | (1,2,2-trimethylpropyl)amino | | |
| 9 | 4-(trifluoromethyl)phenyl | 2-exo-norbornylamino | | |
| 10 | 4-(trifluoromethyl)phenyl | (cyclohexylmethyl)amino | | |
| 11 | 2-(trifluoromethyl)phenyl | 2-(methylpropyl)amino | | |
| 12 | 2-(trifluoromethyl)phenyl | (1,2-dimethylpropyl)amino | | |
| 13 | 2-(trifluoromethyl)phenyl | isopropylamino | | |
| 14 | 2-(trifluoromethyl)phenyl | (1,3-dimethylbutyl)amino | | |
| 15 | 2-(trifluoromethyl)phenyl | (2,2-dimethylpropyl)amino | | |
| 16 | 2-(trifluoromethyl)phenyl | butylamino | 284 | 284 |
| 17 | 2-(trifluoromethyl)phenyl | (4-*tert*-butylcyclohexyl)amino | | |
| 18 | 2-(trifluoromethyl)phenyl | (1,2,2-trimethylpropyl)amino | | |
| 19 | 2-(trifluoromethyl)phenyl | 2-exo-norbornylamino | | |
| 20 | 2-(trifluoromethyl)phenyl | (cyclohexylmethyl)amino | | |
| 21 | 2,3-dichlorophenyl | 2-(methylpropyl)amino | | |
| 22 | 2,3-dichlorophenyl | (1,2-dimethylpropyl)amino | | |
| 23 | 2,3-dichlorophenyl | isopropylamino | | |
| 24 | 2,3-dichlorophenyl | (1,3-dimethylbutyl)amino | | |
| 25 | 2,3-dichlorophenyl | (2,2-dimethylpropyl)amino | 298 | 298 |
| 26 | 2,3-dichlorophenyl | butylamino | | |
| 27 | 2,3-dichlorophenyl | (4-*tert*-butylcyclohexyl)amino | | |
| 28 | 2,3-dichlorophenyl | (1,2,2-trimethylpropyl)amino | | |
| 29 | 2,3-dichlorophenyl | 2-exo-norbornylamino | | |
| 30 | 2,3-dichlorophenyl | (cyclohexylmethyl)amino | | |
| 31 | 2-methoxy-4-nitrophenyl | 2-(methylpropyl)amino | 291 | 291 |
| 32 | 2-methoxy-4-nitrophenyl | (1,2-dimethylpropyl)amino | | |
| 33 | 2-methoxy-4-nitrophenyl | isopropylamino | | |
| 34 | 2-methoxy-4-nitrophenyl | (1,3-dimethylbutyl)amino | | |
| 35 | 2-methoxy-4-nitrophenyl | (2,2-dimethylpropyl)amino | | |
| 36 | 2-methoxy-4-nitrophenyl | butylamino | | |
| 37 | 2-methoxy-4-nitrophenyl | (4-*tert*-butylcyclohexyl)amino | | |
| 38 | 2-methoxy-4-nitrophenyl | (1,2,2-trimethylpropyl)amino | | |
| 39 | 2-methoxy-4-nitrophenyl | 2-exo-norbornylamino | | |
| 40 | 2-methoxy-4-nitrophenyl | (cyclohexylmethyl)amino | 331 | 331 |
| 41 | 2,4-difluorophenyl | 2-(methylpropyl)amino | | |
| 42 | 2,4-difluorophenyl | (1,2-dimethylpropyl)amino | 266 | 266 |
| 43 | 2,4-difluorophenyl | isopropylamino | | |
| 44 | 2,4-difluorophenyl | (1,3-dimethylbutyl)amino | | |
| 45 | 2,4-difluorophenyl | (2,2-dimethylpropyl)amino | | |
| 46 | 2,4-difluorophenyl | butylamino | | |
| 47 | 2,4-difluorophenyl | (4-*tert*-butylcyclohexyl)amino | | |
| 48 | 2,4-difluorophenyl | (1,2,2-trimethylpropyl)amino | | |
| 49 | 2,4-difluorophenyl | 2-exo-norbornylamino | | |
| 50 | 2,4-difluorophenyl | (cyclohexylmethyl)amino | | |
| 51 | 3-chloro-4-fluorophenyl | 2-(methylpropyl)amino | | |
| 52 | 3-chloro-4-fluorophenyl | (1,2-dimethylpropyl)amino | 282 | 282 |
| 53 | 3-chloro-4-fluorophenyl | isopropylamino | | |
| 54 | 3-chloro-4-fluorophenyl | (1,3-dimethylbutyl)amino | | |
| 55 | 3-chloro-4-fluorophenyl | (2,2-dimethylpropyl)amino | | |
| 56 | 3-chloro-4-fluorophenyl | butylamino | | |
| 57 | 3-chloro-4-fluorophenyl | (4-*tert*-butylcyclohexyl)amino | 350 | 350 |
| 58 | 3-chloro-4-fluorophenyl | (1,2,2-trimethylpropyl)amino | | |
| 59 | 3-chloro-4-fluorophenyl | 2-exo-norbomylamino | | |
| 60 | 3-chloro-4-fluorophenyl | (cyclohexylmethyl)amino | | |
| 61 | 4-cyanophenyl | 2-(methylpropyl)amino | | |
| 62 | 4-cyanophenyl | (1,2-dimethylpropyl)amino | 255 | 255 |
| 63 | 4-cyanophenyl | isopropylamino | | |
| 64 | 4-cyanophenyl | (1,3-dimethylbutyl)amino | | |
| 65 | 4-cyanophenyl | (2,2-dimethylpropyl)amino | | |
| 66 | 4-cyanophenyl | butylamino | | |
| 67 | 4-cyanophenyl | (4*-tert*-butylcyclohexyl)amino | | |
| 68 | 4-cyanophenyl | (1,2,2-trimethylpropyl)amino | | |
| 69 | 4-cyanophenyl | 2-exo-norbornylamino | | |
| 70 | 4-cyanophenyl | (cyclohexylmethyl)amino | | |
| 71 | 3,5-bis(trifluoromethyl)phenyl | 2-(methylpropyl)amino | 352 | 352 |
| 72 | 3,5-bis(trifluoromethyl)phenyl | (1,2-dimethylpropyl)amino | | |
| 73 | 3,5-bis(trifluoromethyl)phenyl | isopropylamino | 338 | 338 |
| 74 | 3,5-bis(trifluoromethyl)phenyl | (1,3-dimethylbutyl)amino | 380 | 380 |
| 75 | 3,5-bis(trifluoromethyl)phenyl | (2,2-dimethylpropyl)amino | 366 | 366 |
| 76 | 3,5-bis(trifluoromethyl)phenyl | butylamino | | |
| 77 | 3,5-bis(trifluoromethyl)phenyl | (4-*tert*-butylcyclohexyl)amino | 434 | 434 |
| 78 | 3,5-bis(trifluoromethyl)phenyl | (1,2,2-trimethylpropyl)amino | | |
| 79 | 3,5-bis(trifluoromethyl)phenyl | 2-exo-norbornylamino | 390 | 390 |
| 80 | 3,5-bis(trifluoromethyl)phenyl | (cyclohexylmethyl)amino | | |
| 81 | phenyl | (4-methoxybenzyl)amino | 280 | 280 |
| 82 | 3-fluorophenyl | propylamino | 220 | 220 |
| 83 | 3-fluorophenyl | hexylamino | 262 | 262 |
| 84 | 3-fluorophenyl | propargylamino | 216 | 216 |
| 85 | 3-fluorophenyl | (3-methylbutyl)amino | 248 | 248 |
| 86 | 3-pyridyl | propylamino | 203 | 203 |
| 87 | 3-pyridyl | hexylamino | 245 | 245 |
| 88 | 3-pyridyl | propargylamino | 199 | 199 |
| 89 | 3-pyridyl | (3-methylbutyl)amino | 231 | 231 |
| 90 | 3,4-dichlorophenyl | propylamino | 271 | 271 |
| 91 | 3,4-dichlorophenyl | hexylamino | 313 | 313 |
| 91 | 3,4-dichlorophenyl | propargylamino | 267 | 267 |
| 92 | 3,4-dichlorophenyl | (3-methylbutyl)amino | 299 | 299 |
| 94 | 3,5-bis(trifluoromethyl)phenyl | propylamino | 338 | 338 |
| 95 | 3,5-bis(trifluoromethyl)phenyl | hexylamino | 380 | 380 |
| 96 | 3,5-bis(trifluoromethyl)phenyl | propargylamino | 334 | 334 |
| 97 | 3,5-bis(trifluoromethyl)phenyl | (3-methylbutyl)amino | 366 | 366 |
| 98 | phenyl | phenylamino | 236 | 236 |
| 99 | benzyl | (4-methoxybenzyl)amino | 294 | 294 |
| 100 | phenyl | 1-pyrrolidinyl | 214 | 214 |
| 101 | 3-(trifluoromethyl)phenyl | (3-methylbutyl)amino | 297 | 297 |
| 102 | 4-chloro-3-(trifluoromethyl)-phenyl | (3-methylbutyl)amino | 331 | 331 |
| 103 | 3-acetylphenyl | (3-methylbutyl)amino | 271 | 271 |
| 104 | 2-chloro-5-(trifluoromethyl)-phenyl | (3-methylbutyl)amino | 331 | 331 |
| 105 | 3,4-dicyanophenyl | (3-methylbutyl)amino | 279 | 279 |
| 106 | 4-bromo-2-(trifluoromethyl)-phenyl | (3-methylbutyl)amino | 376 | 376 |
| 107 | 4,6-dimethyl-2-pyrimidinyl | (3-methylbutyl)amino | 259 | 259 |
| 108 | 4-acetylphenyl | (3-methylbutyl)amino | 271 | 271 |
| 109 | 3,5-dichlorophenyl | (3-methylbutyl)amino | 298 | 298 |
| 110 | 3-chloro-4-methylphenyl | (3-methylbutyl)amino | 277 | 277 |
| 111 | 2,5-bis(trifluoromethyl)phenyl | (3-methylbutyl)amino | 366 | 366 |
| 112 | 3,5-dichlorophenyl | (2-methylpropyl)amino | 284 | 284 |
| 113 | 3-chloro-4-methylphenyl | (2-methylpropyl)amino | 263 | 263 |
| 114 | 2,5-bis(trifluoromethyl)phenyl | (2-methylpropyl)amino | 351 | 351 |
| 115 | 3,5-bis(trifluoromethyl)phenyl | (3-phenylpropyl)amino | 413 | 413 |
| 116 | 3,5-bis(trifluoromethyl)phenyl | (2,2,6,6-tetramethyl-4-piperidinyl)amino | 434 | 434 |
| 117 | 3,5-bis(trifluoromethyl)phenyl | N,N-dipropylamino | 379 | 379 |
| 118 | 3,5-bis(trifluoromethyl)phenyl | 2-(4-chlorophenyl)ethylamino | 433 | 433 |
| 119 | 3,5-bis(trifluoromethyl)phenyl | 2-(2-pyridyl)ethylamino | 400 | |
| 120 | 3,5-bis(trifluoromethyl)phenyl | 4-methyl-1-piperidyl | 377 | 377 |
| 121 | 3,5-bis(trifluoromethyl)phenyl | N,N-bis(2-methylpropyl)amino | 407 | 407 |
| 122 | 3,5-bis(trifluoromethyl)phenyl | 1-pyrrolidinyl | 349 | 349 |
| 123 | 3,5-bis(trifluoromethyl)phenyl | 3-(1-imidazolyl)propylamino | 403 | 403 |
| 124 | 3,5-bis(trifluoromethyl)phenyl | N-methyl-N-(3-pyridyl)methylamino | 400 | 400 |
| 125 | 3,5-bis(trifluoromethyl)phenyl | (3-amino-2,2-dimethylpropyl)amino | 380 | 380 |
| 126 | 3,5-bis(trifluoromethyl)phenyl | 3-(2-oxo-1-pyrrolidinyl)propylamino | 420 | 420 |
| 127 | 3,5-bis(trifluoromethyl)phenyl | (4-methoxybenzyl)amino | 415 | 415 |
| 128 | 3,5-bis(trifluoromethyl)phenyl | 3-hydroxy-1-piperidinyl | 379 | |
| 129 | 3,5-bis(trifluoromethyl)phenyl | tetrahydroisoquinolin-1-yl | 411 | 411 |
| 130 | 3,5-bis(trifluoromethyl)phenyl | 2,6-cis-dimethyl-4-morpholinyl | 393 | 393 |
| 131 | 3,5-bis(trifluoromethyl)phenyl | 4-[(3-trifluoromethyl)phenyll-1-piperazinyl | 508 | 508 |
| 132 | 3,5-bis(trifluoromethyl)phenyl | 4-tert-butyl-1-piperidinyl | 419 | 419 |
| 133 | 3,5-bis(trifluoromethyl)phenyl | 1-azepanyl | 377 | 377 |
| 134 | 3,5-bis(trifluoromethyl)phenyl | 4-benzoyl-1-piperidinyl | 467 | 467 |
| 135 | phenyl | tetrahydroisoquinolin-1 -yl | 275 | 275 |
| 136 | phenyl | (4-methylphenyl)amino | 249 | 249 |
| 137 | 3-cyanophenyl | 4-(4-chlorophenyl)-1-piperazinyl | 363 | 363 |
| 138 | 3-acetylphenyl | tetrahydroisoquinolin-1-yl | 317 | 317 |
| 139 | 3-cyanophenyl | N-ethyl-N-phenylamino | 289 | 289 |
| 140 | phenyl | (4-chlorophenyl)amino | 270 | 270 |

## Claims

1. A compound of the general formula I 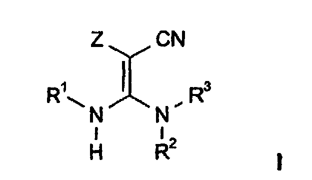 wherein
R¹ is aryl optionally substituted;
R² and R³ are independently hydrogen; alkyl optionally substituted with aryl, heteroaryl, a 5-,6- or 7-membered heterocyclic system, halogen, hydroxy, alkoxy, aryloxy, alkylthio, arylthio, dialkylamino, arylalkylamino or diarylamino; aryl optionally substituted with alkyl, aryl, heteroaryl, halogen, alkoxy, aryloxy, dialkylamino, alkylarylamino, diarylamino, nitro, alkyl-sulfonyl, aryl-sulfonyl, cyano, alkoxycarbonyl or aminocarbonyl; heteroaryl optionally substituted with alkyl, aryl, heteroaryl, halogen, alkoxy, aryloxy, dialkylamino, alkylarylamino, diarylamino, halogen, nitro, alkyl-sulfonyl, aryl-sulfonyl, cyano, alkoxycarbonyl or aminocarbonyl; or
R² and R³ are linked together by -(CH₂)ₙ-, n being 4-7,provided that R² and R³ cannot be hydrogen at the same time;
Z is hydrogen; and
pharmaceutically acceptable salts thereof.

2. A compound according to claim 1 wherein R¹ is optionally substituted phenyl.

3. A compound according to claim 2 wherein R¹ is phenyl substituted by one or two halogen-, perhalomethyl- or cyano-groups.

4. A compound which is selected from the group consisting of: 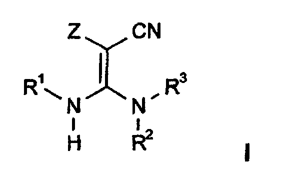
| **No** | **R**^{**1**} | **-NR**^{**2**}**R**^{**3**} | **Z** |
|---|---|---|---|
| 1 | 4-(trifluoromethyl)phenyl | 2-(methylpropyl)amino | H |
| 2 | 4-(trifluoromethyl)phenyl | (1,2-dimethylpropyl)amino | H |
| 3 | 4-(trifluoromethyl)phenyl | isopropylamino | H |
| 4 | 4-(trifluoromethyl)phenyl | (1,3-dimethylbutyl)amino | H |
| 5 | 4-(trifluoromethyl)phenyl | (2,2-dimethylpropyl)amino | H |
| 6 | 4-(trifluoromethyl)phenyl | butylamino | H |
| 7 | 4-(trifluoromethyl)phenyl | *(4-tert*-butylcyclohexyl)amino | H |
| 8 | 4-(trifluoromethyl)phenyl | (1,2,2-trimethylpropyl)amino | H |
| 9 | 4-(trifluoromethyl)phenyl | 2-exo-norbornylamino | H |
| 10 | 4-(trifluoromethyl)phenyl | (cyclohexylmethyl)amino | H |
| 11 | 2-(trifluoromethyl)phenyl | 2-(methylpropyl)amino | H |
| 12 | 2-(trifluoromethyl)phenyl | (1,2-dimethylpropyl)amino | H |
| 13 | 2-(trifluoromethyl)phenyl | isopropylamino | H |
| 14 | 2-(trifluoromethyl)phenyl | (1,3-dimethylbutyl)amino | H |
| 15 | 2-(trifluoromethyl)phenyl | (2,2-dimethylpropyl)amino | H |
| 16 | 2-(trifluoromethyl)phenyl | butylamino | H |
| 17 | 2-(trifluoromethyl)phenyl | (4-*tert*-butylcyclohexyl)amino | H |
| 18 | 2-(trifluoromethyl)phenyl | (1,2,2-trimethylpropyl)amino | H |
| 19 | 2-(trifluoromethyl)phenyl | 2-exo-norbornylamino | H |
| 20 | 2-(trifluoromethyl)phenyl | (cyclohexylmethyl)amino | H |
| 21 | 2,3-dichlorophenyl | 2-(methylpropyl)amino | H |
| 22 | 2,3-dichlorophenyl | (1,2-dimethylpropyl)amino | H |
| 23 | 2,3-dichlorophenyl | isopropylamino | H |
| 24 | 2,3-dichlorophenyl | (1,3-dimethylbutyl)amino | H |
| 25 | 2,3-dichlorophenyl | (2,2-dimethylpropyl)amino | H |
| 26 | 2,3-dichlorophenyl | butylamino | H |
| 27 | 2,3-dichlorophenyl | (4-*tert*-butylcyclohexyl)amino | H |
| 28 | 2,3-dichlorophenyl | (1,2,2-trimethylpropyl)amino | H |
| 29 | 2,3-dichlorophenyl | 2-exo-norbornylamino | H |
| 30 | 2,3-dichlorophenyl | (cyclohexylmethyl)amino | H |
| 31 | 2-methoxy-4-nitrophenyl | 2-(methylpropyl)amino | H |
| 32 | 2-methoxy-4-nitrophenyl | (1,2-dimethylpropyl)amino | H |
| 33 | 2-methoxy-4-nitrophenyl | isopropylamino | H |
| 34 | 2-methoxy-4-nitrophenyl | (1,3-dimethylbutyl)amino | H |
| 35 | 2-methoxy-4-nitrophenyl | (2,2-dimethylpropyl)amino | H |
| 36 | 2-methoxy-4-nitrophenyl | butylamino | H |
| 37 | 2-methoxy-4-nitrophenyl | (4-*tert*-butylcydohexyl)amino | H |
| 38 | 2-methoxy-4-nitrophenyl | (1,2,2-trimethylpropyl)amino | H |
| 39 | 2-methoxy-4-nitrophenyl | 2-exo-norbornylamino | H |
| 40 | 2-methoxy-4-nitrophenyl | (cyclohexylmethyl)amino | H |
| 41 | 2,4-difluorophenyl | 2-(methylpropyl)amino | H |
| 42 | 2,4-difluorophenyl | (1,2-dimethylpropyl)amino | H |
| 43 | 2,4-difluorophenyl | isopropylamino | H |
| 44 | 2,4-difluorophenyl | (1,3-dimethylbutyl)amino | H |
| 45 | 2,4-difluorophenyl | (2,2-dimethylpropyl)amino | H |
| 46 | 2,4-difluorophenyl | butylamino | H |
| 47 | 2,4-difluorophenyl | (4-*tert*-butylcyclohexyl)amino | H |
| 48 | 2,4-difluorophenyl | (1,2,2-trimethylpropyl)amino | H |
| 49 | 2,4-difluorophenyl | 2-exo-norbomylamino | H |
| 50 | 2,4-difluorophenyl | (cyclohexylmethyl)amino | H |
| 51 | 3-chloro-4-fluorophenyl | 2-(methylpropyl)amino | H |
| 52 | 3-chloro-4-fluorophenyl | (1,2-dimethylpropyl)amino | H |
| 53 | 3-chloro-4-fluorophenyl | isopropylamino | H |
| 54 | 3-chloro-4-fluorophenyl | (1,3-dimethylbutyl)amino | H |
| 55 | 3-chloro-4-fluorophenyl | (2,2-dimethylpropyl)amino | H |
| 56 | 3-chloro-4-fluorophenyl | butylamino | H |
| 57 | 3-chloro-4-fluorophenyl | (4-*tert*-butylcyclohexyl)amino | H |
| 58 | 3-chloro-4-fluorophenyl | (1,2,2-trimethylpropyl)amino | H |
| 59 | 3-chloro-4-fluorophenyl | 2-exo-norbomylamino | H |
| 60 | 3-chloro-4-fluorophenyl | (cyclohexylmethyl)amino | H |
| 61 | 4-cyanophenyl | 2-(methylpropyl)amino | H |
| 62 | 4-cyanophenyl | (1,2-dimethylpropyl)amino | H |
| 63 | 4-cyanophenyl | isopropylamino | H |
| 64 | 4-cyanophenyl | (1,3-dimethylbutyl)amino | H |
| 65 | 4-cyanophenyl | (2,2-dimethylpropyl)amino | H |
| 66 | 4-cyanophenyl | butylamino | H |
| 67 | 4-cyanophenyl | (4-*tert*-butylcydohexyl)amino | H |
| 68 | 4-cyanophenyl | (1,2,2-trimethylpropyl)amino | H |
| 69 | 4-cyanophenyl | 2-exo-norbomylamino | H |
| 70 | 4-cyanophenyl | (cyclohexylmethyl)amino | H |
| 71 | 3,5-bis(trifluoromethyl)phenyl | 2-(methylpropyl)amino | H |
| 72 | 3,5-bis(trifluoromethyl)phenyl | (1,2-dimethylpropyl)amino | H |
| 73 | 3,5-bis(trifluoromethyl)phenyl | isopropylamino | H |
| 74 | 3,5-bis(trifluoromethyl)phenyl | (1,3-dimethylbutyl)amino | H |
| 75 | 3,5-bis(trifluoromethyl)phenyl | (2,2-dimethylpropyl)amino | H |
| 76 | 3,5-bis(trifluoromethyl)phenyl | butylamino | H |
| 77 | 3,5-bis(trifluoromethyl)phenyl | (4-*tert*-butylcyclohexyl)amino | H |
| 78 | 3,5-bis(trifluoromethyl)phenyl | (1,2,2-trimethylpropyl)amino | H |
| 79 | 3,5-bis(trifluoromethyl)phenyl | 2-exo-norbomylamino | H |
| 80 | 3,5-bls(trifluoromethyl)phenyl | (cyclohexylmethyl)amino | H |
| 81 | phenyl | (4-methoxybenzyl)amino | H |
| 82 | 3-fluorophenyl | propylamino | H |
| 83 | 3-fluorophenyl | hexylamino | H |
| 84 | 3-fluorophenyl | propargylamino | H |
| 85 | 3-fluorophenyl | (3-methylbutyl)amino | H |
| 86 | 3-pyridyl | propylamino | H |
| 87 | 3-pyridyl | hexylamino | H |
| 88 | 3-pyridyl | propargylamino | H |
| 89 | 3-pyridyl | (3-methylbutyl)amino | H |
| 90 | 3,4-dichlorophenyl | propylamino | H |
| 91 | 3,4-dichlorophenyl | hexylamino | H |
| 91 | 3,4-dichlorophenyl | propargylamino | H |
| 92 | 3,4-dichlorophenyl | (3-methylbutyl)amino | H |
| 94 | 3,5-bis(trifluoromethyl)phenyl | propylamino | H |
| 95 | 3,5-bis(trifluoromethyl)phenyl | hexylamino | H |
| 96 | 3,5-bis(trifluoromethyl)phenyl | propargylamino | H |
| 97 | 3,5-bis(trifluoromethyl)phenyl | (3-methylbutyl)amino | H |
| 98 | phenyl | phenylamino | H |
| 99 | benzyl, | (4-methoxybenzyl)amino | H |
| 100 | phenyl | 1-pyrrolidinyl | H |
| 101 | 3-(trifluoromethyl)phenyl | (3-methylbutyl)amino | H |
| 102 | 4-chloro-3-(trifluoromethyl)-phenyl | (3-methylbutyl)amino | H |
| 103 | 3-acetylphenyl | (3-methylbutyl)amino | H |
| 104 | 2-chloro-5-(trifluoromethyl)-phenyl | (3-methylbutyl)amino | H |
| 105 | 3,4-dicyanophenyl | (3-methylbutyl)amino | H |
| 106 | 4-bromo-2-(trifluoromethyl)-phenyl | (3-methylbutyl)amino | H |
| 107 | 4,6-dimethyl-2-pyrimidinyl | (3-methylbutyl)amino | H |
| 108 | 4-acetylphenyl | (3-methylbutyl)amino | H |
| 109 | 3,5-dichlorophenyl | (3-methylbutyl)amino | H |
| 110 | 3-chloro-4-methylphenyl | (3-methylbutyl)amino | H |
| 111 | 2,5-bis(trifluoromethyl)phenyl | (3-methylbutyl)amino | H |
| 112 | 3,5-dichlorophenyl | (2-methylpropyl)amino | H |
| 113 | 3-chloro-4-methylphenyl | (2-methylpropyl)amino | H |
| 114 | 2,5-bis(trifluoromethyl)phenyl | (2-methylpropyl)amino | H |
| 115 | 3,5-bis(trifluoromethyl)phenyl | (3-phenylpropyl)amino | H |
| 116 | 3,5-bis(trifluoromethyl)phenyl | (2,2,6,6-tetramethyl-4-piperidinyl)amino | H |
| 117 | 3,5-bis(trifluoromethyl)phenyl | N,N-dipropylamino | H |
| 118 | 3,5-bis(trifluoromethyl)phenyl | 2-(4-chlorophenyl)ethylamino | H |
| 119 | 3,5-bis(trifluoromethyl)phenyl | 2-(2-pyridyl)ethylamino | H |
| 120 | 3,5-bis(trifluoromethyl)phenyl | 4-methyl-1-piperidyl | H |
| 121 | 3,5-bis(trifluoromethyl)phenyl | N,N-bis(2-methylpropyl)amino | H |
| 122 | 3,5-bis(trifluoromethyl)phenyl | 1-pyrrolidinyl | H |
| 123 | 3,5-bis(trifluoromethyl)phenyl | 3-(1-imidazolyl)propylamino | H |
| 124 | 3,5-bis(trifluoromethyl)phenyl | N-methyl-N-(3-pyridyl)methylamino | H |
| 125 | 3,5-bis(trifluoromethyl)phenyl | (3-amino-2,2-dimethylpropyl)amino | H |
| 126 | 3,5-bit(trifluoromethyl)phenyl | 3-(2-oxo-1-pyrrolidinyl)propylamino | H |
| 127 | 3,5-bis(trifluoromethyl)phenyl | (4-methoxybenzyl)amino | H |
| 128 | 3,5-bis(trifluoromethyl)phenyl | 3-hydroxy-1-piperidinyl | H |
| 129 | 3,5-bis(trifluoromethyl)phenyl | tetrahydroisoquinolin-1-yl | H |
| 130 | 3,5-bis(trifluoromethyl)phenyl | 2,6-cis-dimethyl-4-morpholinyl | H |
| 131 | 3,5-bis(trifluoromethyl)phenyl | 4-[(3-trifluoromethyl)phenyl]-1-piperazinyl | H |
| 132 | 3,5-bis(trifluoromethyl)phenyl | 4-*tert*-butyl-1-piperidinyl | H |
| 133 | 3,5-bis(trifluoromethyl)phenyl | 1-azepanyl | H |
| 134 | 3,5-bis(trifluoromethyl)phenyl | 4-benzoyl-1-piperidinyl | H |
| 135 | phenyl | tetrahydroisoquinotin-1-yl | H |
| 136 | phenyl | (4-methylphenyl)amino | H |
| 137 | 3-cyanophenyl | 4-(4-chlorophenyl)-1-piperazinyl | H |
| 138 | 3-acetylphenyl | tetrahydroisoquinolin-1-yl | H |
| 139 | 3,5-bis(trifluoromethyl)phenyl | 3-(2-oxo-1-azepanyl)propylamino | H |
| 140 | 3-cyanophenyl | N-ethyl-N-phenylamino | H |
| 141 | phenyl | (4-chlorophenyl)amino | H |
and pharmaceutically acceptable salts thereof.

5. Compounds according to any one of the preceding claims for use as potassium channel openers.

6. A pharmaceutical composition comprising a compound according to any of the claim 1 - 4 or a pharmaceutical acceptable salt thereof with a pharmaceutically acceptable acid or base, or any optical isomer or mixture of optical isomers, including a racemic mixture, or any tautomeric form together with one or more pharmaceutically acceptable carriers or diluents.

7. A pharmaceutical composition for use in the treatment of diseases of the endocrinological system such as diabetes comprising a compound according to any of the claims 1 - 4 or a pharmaceutical acceptable salt thereof with a pharmaceutically acceptable acid or base, or any optical isomer or mixture of optical isomers, including a racemic mixture, or any tautomeric form together with one or more pharmaceutically acceptable carriers or diluents.

8. The pharmaceutical composition according to claim 6 or 7 in the form of an oral dosage unit or parenteral dosage unit.

9. A pharmaceutical composition according to claims 6 - 8 wherein said compound is administered as a dose in a range from about 0.05 mg to 1000 mg, preferably from about 0.1 mg to 500 mg and especially in the range from 50 mg to 200 mg per day.

10. A compound according to any one of the claims 1 - 4 or a pharmaceutically acceptable salt thereof with a pharmaceutically acceptable acid or base, or any optical isomer or mixture of optical isomers, including a racemic mixture, or any tautomeric form for therapeutical use.

11. A compound according to any one of the claims 1 - 4 or a pharmaceutically acceptable salt thereof with a pharmaceutically acceptable acid or base, or any optical isomer or mixture of optical isomers, including a racemic mixture, or any tautomeric form for therapeutical use in the treatment or prevention of diseases of the endocrinological system, such as diabetes.

12. The use of a compound according to any of the claims 1 - 4 or a pharmaceutically acceptable salt thereof with a pharmaceutically acceptable add or base, or any optical isomer or mixture of optical isomers, including a racemic mixture, or any tautomeric form for preparing a medicament.

13. The use of a compound according to any one of the claims 1 - 4 or a pharmaceutically acceptable salt thereof with a pharmaceutically acceptable add or base, or any optical isomer or mixture of optical isomers, induding a racemic mixture, or any tautomeric form for the preparation of a medicament for the treatment or prevention of diseases of the endocrinoiogical system, such as diabetes.

14. A process for the manufacture of a medicament to be used in the treatment or prevention of diseases of the endocrinological system, such as diabetes which process comprising bringing a compound of formula I according to any of the claims 1 - 4 or a pharmaceutically acceptable salt thereof into a galenic dosage form.

15. A compound of the general formula **II** 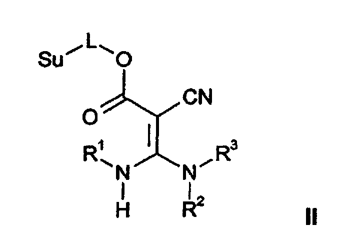 wherein
• Su is a substrate referring to any insoluble or partially insoluble material, to which compounds may be covalently attached,
• L is a chemical bond or linker which is defined as a molecule with at least two reactive sites, which permit its covalent attachment to other molecules or to a substrate linker,
• R¹, R²and R³ are as defined in claim 1.

16. A compound according to claim 15 useful for screening for potassium channel openers.

17. A method for preparing a compound according to claim 1 comprising the steps of:
a) acylation of a substrate-bound alcohol of the formula Su-L-O-H, wherein Su and L are as defined in claim 15, with a cyanoacetic acid derivative of the general structure NC-CH₂-COX, wherein X is a hydroxy group or a leaving group;
b) reaction of the resulting substrate-bound intermediate of the formula Su-L-O-CO-CH₂-CN wherein Su and L are as defined in claim 15, with an aliphatic or aromatic isothiocyanate of the general structure R¹-NCS wherein R¹ is as defined in claim 15, in the presence of a base;
c) reaction of the resulting substrate-bound intermediate of the formula Su-L-O-CO-CH(CN)-C(S)(NHR¹) with an amine of the general structure R²R³NH, wherein R² and R³ is as defined in claim 1, in the presence of a desulfurizing agent, in order to prepare a compound of formula **II**,
d) subjection of the resulting substrate-bound compound of formula **II** to cleavage conditions in order to prepare a compound of formula **I**.

18. The method according to claim 17 further comprising the step of screening the final product of formula I directly against a specific receptor or enzyme.

19. A method for preparing a compound according to claim 15 comprising the steps of:
a) acylation of a substrate-bound alcohol of the formula Su-L-O-H, wherein Su and L are as defined in claim 15, with a cyanoacetic acid derivative of the general structure NC-CH₂-COX, wherein X is a hydroxy group or a leaving group;
b) reaction of the resulting substrate-bound intermediate of the formula Su-L-O-CO-CH₂-CN wherein Su and L are as defined in claim 15, with an aliphatic or aromatic isothiocyanate of the general structure R¹-NCS wherein R¹ is as defined in claim 15, in the presence of a base;
c) reaction of the resulting substrate-bound intermediate of the formula Su-L-O-CO-CH(CN)-C(S)(NHR¹) with an amine of the general structure R²R³NH, wherein R² and R³ is as defined in claim 1, in the presence of a desulfurizing reagent, in order to prepare a compound of formula II.

20. The method according to claim 19 further comprising the step of screening the final product of formula II directly against a specific receptor or enzyme.

21. The method according to claim 15 to 20 wherein the desulfurizing reagent is EDC.

22. The method according to claim 15 of 21 wherein the base for the reaction of the isothiocyanate with the cyanoacetaic acid ester is a tertiary amine.

23. The method according to claim 15 to 22 wherein the cyanoacetic acid derivative is the symmetrical anhydride.

24. An array comprising m different compounds of formula **I**, at selected known positions in m containers, wherein m is an integer equal to or greater than 2.

25. An array comprising m different compounds of formula **II**, wherein m is an integer equal to or greater than 2, at selected known positions on one or more substrates.

26. The array according to claim 24 or 25, wherein m is between 60 to 100, preferably 80.

27. An array comprising one compound of formula **I** or n different compounds of formula **I**, wherein n is an integer equal to or greater than 2, at selected known positions in m containers, or at selected known positions on a substrate, and one compound of formula **II** or m-n different compounds of formula **II**, wherein m is an integer equal to or greater than 2, and m>n, at selected known positions on one or more substrates.

28. The array according to claim 27 wherein m is between 60 to 100, preferably 80.

29. An array comprising p different mixtures of compounds of formula **I**, at selected known positions in p containers, wherein p is an integer equal to or greater than 2.

30. An array comprising p different mixtures of compounds of formula **II** wherein p is an integer equal to or greater than 2, at selected known positions on one or more substrates.

31. The array according to claim 29 or 30, wherein p is between 60 to 100, preferably 80.

32. An array comprising one mixture of compounds of formula **I** or r different mixtures of compounds of formula **I**, wherein r is an integer equal to or greater than 2, at selected known positions in p containers, or at selected known positions on a substrate, and one mixture of compounds of formula **II**, or p-r different mixtures of compounds of formula **II**, wherein p is an integer equal to or greater than 2, and p>r, at selected known positions on one or more substrates.

33. The array according to claim 32, wherein p is between 60 to 100, preferably 80.

34. A method for preparing the array according to claim 25 or 26 comprising, carrying out at selected known positions on one or more substrate(s) the steps of:
a) simultaneous acylation of each and every single substrate-bound alcohol of the formula Su-L-OH, wherein Su and L are as defined in claim 15, with a cyanoacetic acid derivative of the general structure NC-CH₂-COX, wherein X is a hydroxy group or a leaving group;
b) reaction of each and every one of the resulting substrate-bound esters of the formula Su-L-O-CO-CH₂-CN wherein Su and L are as defined in claim 15, with an isothiocyanate of the general structure R¹-NCS wherein R¹ is as defined in claim 15, in the presence of a base;
c) alkylation of each and every one of the resulting substrate-bound intermediates of the formula Su-L-O-CO-CH(CN)-C(S)(NHR¹) with an amine of the general structure R²R³NH, wherein R² and R³ are as defined in claim 1, in the presence of a desulfurizing reagent, in order to prepare a compound of formula II, attached to one or more substrate(s).

35. A method for preparing the array according to claim 28 or 30, the method of claim 34 further comprising the step of:
d) subjection of the resulting m substrate-bound compounds of formula **II** to cleavage conditions in order to prepare m compounds of formula **I**, at selected known positions in m containers, wherein m is an integer equal to or greater than 2.

36. A method for preparing the array according to claim 27 or 28, the method of claim 34 further comprising the step of:
d) subjection of the resulting m substrate-bound compounds of formula **II** to cleavage conditions in order to prepare n compounds of formula **I**, and m-n compounds of formula **II**, at selected known positions in m containers, or at selected known positions on a substrate.

37. The method according to any one of the claims 34, 35 or 36 wherein the base for the reaction of the isothiocyanate with the cyanoacetic acid in claim 34, step b) is a *tert*iary amine.

38. The method according to any one of the claims 34 to 37 wherein the cyanoacetic acid derivative is the symmetrical anhydride.

39. The method according to any one of the claims 34 to 38 further comprising screening the final products directly against a specific receptor or enzyme.

40. The array of compounds of the formula **I,** according to claim 24 or 26 wherein R¹ is phenyl, 4-trifluoromethylphenyl, 2-trifluoromethylphenyl, 2,3-dichlorophenyl, 3-chloro-4-fluorophenyl, 2-methoxy-4-nitrophenyl, 2,4-difluorophenyl, 4-cyanophenyl, 3,5-bis(trifluoromethyl)phenyl, 3-fluorophenyl, 3-pyridyl, 3,4-dichlorophenyl or benzyl.

41. The array of compounds of the formula I, according to any one of the claims 24, 26 or 39 wherein R² is 2-methylpropyl, 1,2-dimethylpropyl, isopropyl, 1,3-dimethylbutyl, 2,2-dimethylpropyl, butyl, 4-*tert*-butylcyclohexyl, 1,2,2-trimethylpropyl, exo-2-norbornyl and cyclohexylmethyl, 4-methoxybenzyl, phenyl, propyl, hexyl, propargyl or 3-methylbutyl; or
R² and R³ are linked together by -(CH₂)ₙ-, n being 4-7.

42. Use of an array according to any one of the claim 24 to 33 for screening compounds of formula **I** against specific receptors or enzymes.

43. Use of an array according to any one of the claim 24 to 33 for screening compounds of formula **I** against potassium channels.

## Patentansprüche

1. Verbindung der allgemeinen Formel I 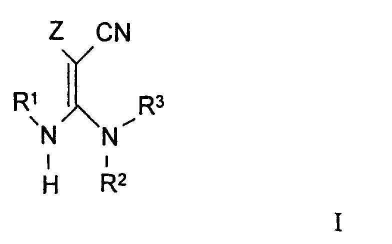 wobei
R¹ Aryl, wahlweise substituiert, ist;
R² und R³ unabhängig voneinander Wasserstoff; Alkyl, wahlweise substituiert durch Aryl, Heteroaryl, ein 5-, 6- oder 7-gliedriges heterozyklisches System, Halogen, Hydroxy, Alkoxy, Aryloxy, Alkylthio, Arylthio, Dialkylamino, Arylakylamino oder Diarylamino; Aryl, wahlweise substituiert durch Alkyl, Aryl, Heteroaryl, Halogen, Alkoxy, Aryloxy, Dialkylamino, Alkylarylamino, Diarylamino, Nitro, Alkyl-sulfonyl, Aryl-sulfonyl, Cyano, Alkoxycarbonyl oder Aminocarbonyl; Heteroaryl, wahlweise substituiert durch Alkyl, Aryl, Heteroaryl, Halogen, Alkoxy, Aryloxy, Dialkylamino, Alkylarylamino, Diarylamino, Halogen, Nitro, Alkyl-sulfonyl, Aryl-sulfonyl, Cyano, Alkoxycarbonyl oder Aminocarbonyl; sind oder
R² und R³ miteinander verknüpft sind durch -(CH₂)ₙ-, wobei n 4-7 ist, vorausgesetzt, dass R² und R³ nicht zur gleichen Zeit Wasserstoff sein können;
Z Wasserstoff ist; und
pharmazeutisch geeignete Salze davon.

2. Verbindung nach Anspruch 1, wobei R¹ wahlweise substituiertes Phenyl ist.

3. Verbindung nach Anspruch 2, wobei R¹ Phenyl, substituiert durch eine oder zwei Halogen-, Perhalomethyl- oder Cyano-Gruppen, ist.

4. Verbindung, welche ausgewählt ist aus der Grupp7e bestehend aus: 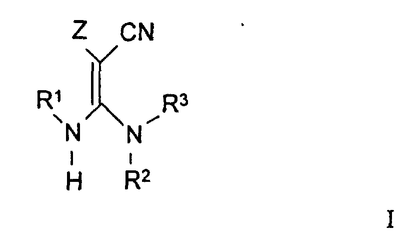
| **Nr.** | **R**^{**1**} | **-NR**^{**2**}**R**^{**3**} | **Z** |
|---|---|---|---|
| 1 | 4-(Trifluormethyl)phenyl | 2-(Methylpropyl)amino | H |
| 2 | 4-(Trifluormethyl)phenyl | (1,2-Dimethylpropyl)amino | H |
| 3 | 4-(Trifluormethyl)phenyl | Isopropylamino | H |
| 4 | 4-(Trifluormethyl)phenyl | (1,3-Dimethylbutyl)amino | H |
| 5 | 4-(Trifluormethyl)phenyl | (2,2-Dimethylpropyl)amino | H |
| 6 | 4-(Trifluormethyl)phenyl | Butylamino | H |
| 7 | 4-(Trifluormethyl)phenyl | (4-*tert*-Butylcyclohexyl)amino | H |
| 8 | 4-(Trifluormethyl)phenyl | (1,2,2-Trimethylpropyl)amino | H |
| 9 | 4-(Trifluormethyl)phenyl | 2-exo-Norbornylamino | H |
| 10 | 4-(Trifluormethyl)phenyl | (Cyclohexylmethyl)amino | H |
| 11 | 2-(Trifluormethyl)phenyl | 2-(Methylpropyl)amino | H |
| 12 | 2-(Trifluormethyl)phenyl | (1,2-Dimethylpropyl)amino | H |
| 13 | 2-(Trifluormethyl)phenyl | Isopropylamino | H |
| 14 | 2-(Trifluormethyl)phenyl | (1,3-Dimethylbutyl)amino | H |
| 15 | 2-(Trifluormethyl)phenyl | (2,2-Dimethylpropyl)amino | H |
| 16 | 2-(Trifluormethyl)phenyl | Butylamino | H |
| 17 | 2-(Trifluormethyl)phenyl | (4-*tert*-Butylcyclohexyl)amino | H |
| 18 | 2-(Trifluormethyl)phenyl | (1,2,2-Trimethylpropyl)amino | H |
| 19 | 2-(Trifluormethyl)phenyl | 2-exo-Norbornylamino | H |
| 20 | 2-(Trifluormethyl)phenyl | (Cyclohexylmethyl)amino | H |
| 21 | 2,3-Dichlorphenyl | 2-(Methylpropyl)amino | H |
| 22 | 2,3-Dichlorphenyl | (1,2-Dimethylpropyl)amino | H |
| 23 | 2,3-Dichlorphenyl | Isopropylamino | H |
| 24 | 2,3-Dichlorphenyl | (1,3-Dimethylbutyl)amino | H |
| 25 | 2,3-Dichlorphenyl | (2,2-Dimethylpropyl)amino | H |
| 26 | 2,3-Dichlorphenyl | Butylamino | H |
| 27 | 2,3-Dichlorphenyl | (4-*tert*-Butylcyclohexyl)amino | H |
| 28 | 2,3-Dichlorphenyl | (1,2,2-Trimethylpropyl)amino | H |
| 29 | 2,3-Dichlorphenyl | 2-exo-Norbornylamino | H |
| 30 | 2,3-Dichlorphenyl | (Cyclohexylmethyl)amino | H |
| 31 | 2-Methoxy-4-nitrophenyl | 2-(Methylpropyl)amino | H |
| 32 | 2-Methoxy-4-nitrophenyl | (1,2-Dimethylpropyl)amino | H |
| 33 | 2-Methoxy-4-nitrophenyl | Isopropylamino | H |
| 34 | 2-Methoxy-4-nitrophenyl | (1,3-Dimethylbutyl)amino | H |
| 35 | 2-Methoxy-4-nitrophenyl | (2,2-Dimethylpropyl)amino | H |
| 36 | 2-Methoxy-4-nitrophenyl | Butylamino | H |
| 37 | 2-Methoxy-4-nitrophenyl | (4-*tert*-Butylcyclohexyl)amino | H |
| 38 | 2-Methoxy-4-nitrophenyl | (1,2,2-Trimethylpropyl)amino | H |
| 39 | 2-Methoxy-4-nitrophenyl | 2-exo-Norbornylamino | H |
| 40 | 2-Methoxy-4-nitrophenyl | (Cyclohexylmethyl)amino | H |
| 41 | 2,4-Difluorphenyl | 2-(Methylpropyl)amino | H |
| 42 | 2,4-Difluorphenyl | (1,2-Dimethylpropyl)amino | H |
| 43 | 2,4-Difluorphenyl | Isopropylamino | H |
| 44 | 2,4-Difluorphenyl | (1,3-Dimethylbutyl)amino | H |
| 45 | 2,4-Difluorphenyl | (2,2-Dimethylpropyl)amino | H |
| 46 | 2,4-Difluorphenyl | Butylamino | H |
| 47 | 2,4-Difluorphenyl | (4-tert-Butylcyclohexyl)amino | H |
| 48 | 2,4-Difluorphenyl | (1,2,2-Trimethylpropyl)amino | H |
| 49 | 2,4-Difluorphenyl | 2-exo-Norbornylamino | H |
| 50 | 2,4-Difluorphenyl | (Cyclohexylmethyl)amino | H |
| 51 | 3-Chlor-4-fluorphenyl | 2-(Methylpropyl)amino | H |
| 52 | 3-Chlor-4-fluorphenyl | (1,2-Dimethylpropyl)amino | H |
| 53 | 3-Chlor-4-fluorphenyl | Isopropylamino | H |
| 54 | 3-Chlor-4-fluorphenyl | (1,3-Dimethylbutyl)amino | H |
| 55 | 3-Chlor-4-fluorphenyl | (2,2-Dimethylpropyl)amino | H |
| 56 | 3-Chlor-4-fluorphenyl | Butylamino | H |
| 57 | 3-Chlor-4-fluorphenyl | (4-tert-Butylcyclohexyl)amino | H |
| 58 | 3-Chlor-4-fluorphenyl | (1,2,2-Trimethylpropyl)amino | H |
| 59 | 3-Chlor-4-fluorphenyl | 2-exo-Norbornylamino | H |
| 60 | 3-Chlor-4-fluorphenyl | (Cyclohexylmethyl)amino | H |
| 61 | 4-Cyanophenyl | 2-(Methylpropyl)amino | H |
| 62 | 4-Cyanophenyl | (1,2-Dimethylpropyl)amino | H |
| 63 | 4-Cyanophenyl | Isopropylamino | H |
| 64 | 4-Cyanophenyl | (1,3-Dimethylbutyl)amino | H |
| 65 | 4-Cyanophenyl | (2,2-Dimethylpropyl)amino | H |
| 66 | 4-Cyanophenyl | Butylamino | H |
| 67 | 4-Cyanophenyl | (4-tert-Butylcyclohexyl)amino | H |
| 68 | 4-Cyanophenyl | (1,2,2-Trimethylpropyl)amino | H |
| 69 | 4-Cyanophenyl | 2-exo-Norbornylamino | H |
| 70 | 4-Cyanophenyl | (Cyclohexylmethyl)amino | H |
| 71 | 3,5-bis(Trifluormethyl)phenyl | 2-(Methylpropyl)amino | H |
| 72 | 3,5-bis(Trifluormethyl)phenyl | (1,2-Dimethylpropyl)amino | H |
| 73 | 3,5-bis(Trifluormethyl)phenyl | Isopropylamino | H |
| 74 | 3,5-bis(Trifluormethyl)phenyl | (1,3-Dimethylbutyl)amino | H |
| 75 | 3,5-bis(Trifluormethyl)phenyl | (2,2-Dimethylpropyl)amino | H |
| 76 | 3,5-bis(Trifluormethyl)phenyl | Butylamino | H |
| 77 | 3,5-bis(Trifluormethyl)phenyl | (4-*tert*-Butylcyclohexyl)amino | H |
| 78 | 3,5-bis(Trifluormethyl)phenyl | (1,2,2-Trimethylpropyl)amino | H |
| 79 | 3,5-bis(Trifluormethyl)phenyl | 2-exo-Norbornylamino | H |
| 80 | 3,5-bis(Trifluormethyl)phenyl | (Cyclohexylmethyl)amino | H |
| 81 | Phenyl | 2-(Methylpropyl)amino | H |
| 82 | 3-Fluorphenyl | Propylamino | H |
| 83 | 3-Fluorphenyl | Hexylamino | H |
| 84 | 3-Fluorphenyl | Propargylamino | H |
| 85 | 3-Fluorphenyl | (3-Methylbutyl)amino | H |
| 86 | 3-Pyridyl | Propylamino | H |
| 87 | 3-Pyridyl | Hexylamino | H |
| 88 | 3-Pyridyl | Propargylamino | H |
| 89 | 3-Pyridyl | (3-Methylbutyl)amino | H |
| 90 | 3,4-Dichlorphenyl | Propylamino | H |
| 91 | 3,4-Dichlorphenyl | Hexylamino | H |
| 91 | 3,4-Dichlorphenyl | Propargylamino | H |
| 92 | 3,4-Dichlorphenyl | (3-Methylbutyl)amino | H |
| 94 | 3,5-bis(Trifluormethyl)phenyl | Propylamino | H |
| 95 | 3,5-bis(Trifluormethyl)phenyl | Hexylamino | H |
| 96 | 3,5-bis(Trifluormethyl)phenyl | Propargylamino | H |
| 97 | 3,5-bis(Trifluormethyl)phenyl | (3-Methylbutyl)amino | H |
| 98 | Phenyl | Phenylamino | H |
| 99 | Benzyl | (4-Methoxybenzyl)amino | H |
| 100 | Phenyl | 1-Pyrrolidinyl | H |
| 101 | 3-(Trifluormethyl)phenyl | (3-Methylbutyl)amino | H |
| 102 | 4-Chlor-3-(trifluormethyl)phenyl | (3-Methylbutyl)amino | H |
| 103 | 3-Acetylphenyl | (3-Methylbutyl)amino | H |
| 104 | 2-Chlor-5-(trifluormethyl)phenyl | (3-Methylbutyl)amino | H |
| 105 | 3,4-Dicyanphenyl | (3-Methylbutyl)amino | H |
| 106 | 4-Brom-2-(trifluormethyl)phenyl | (3-Methylbutyl)amino | H |
| 107 | 4,6-Dimethyl-2-pyrimidinyl | (3-Methylbutyl)amino | H |
| 108 | 4-Acetylphenyl | (3-Methylbutyl)amino | H |
| 109 | 3,5-Dichlorphenyl | (3-Methylbutyl)amino | H |
| 110 | 3-Chlor-4-methylphenyl | (3-Methylbutyl)amino | H |
| 111 | 2,5-bis(Trifluormethyl)phenyl | (3-Methylbutyl)amino | H |
| 112 | 3,5-Dichlorphenyl | (2-Methylpropyl)amino | H |
| 113 | 3-Chlor-4-Methylphenyl | (2-Methylpropyl)amino | H |
| 114 | 2,5-bis(Trifluormethyl)phenyl | (2-Methylpropyl)amino | H |
| 115 | 3,5-bis(Trifluormethyl)phenyl | (3-Phenylpropyl)amino | H |
| 116 | 3,5-bis(Trifluormethyl)phenyl | (2,2,6,6-Tetramethyl-4-piperidinyl)amino | H |
| 117 | 3,5-bis(Trifluormethyl)phenyl | N,N-Dipropylamino | H |
| 118 | 3,5-bis(Trifluormethyl)phenyl | 2-(4-Chlorphenyl)ethylamino | H |
| 119 | 3,5-bis(Trifluormethyl)phenyl | 2-(2-Pyridyl)ethylamino | H |
| 120 | 3,5-bis(Trifluormethyl)phenyl | 4-Methyl-1-piperidyl | H |
| 121 | 3,5-bis(Trifluormethyl)phenyl | N,N-bis(2-Methylpropyl)amino | H |
| 122 | 3,5-bis(Trifluormethyl)phenyl | 1-Pyrrolidinyl | H |
| 123 | 3,5-bis(Trifluonnethyl)phenyl | 3-(1-Imidazolyl)propylamino | H |
| 124 | 3,5-bis(Trifluormethyl)phenyl | N-Methyl-N-(3-pyridyl)methylamino | H |
| 125 | 3,5-bis(Trifluormethyl)phenyl | (3-Amino-2,2-dimethylpropyl)amino | H |
| 126 | 3,5-bis(Trifluormethyl)phenyl | 3-(2-Oxo-1-pyrrolidinyl)propylamino | H |
| 127 | 3,5-bis(Trifluormethyl)phenyl | (4-Methoxybenzyl)amino | H |
| 128 | 3,5-bis(Trifluormethyl)phenyl | 3-Hydroxy-1-piperidinyl | H |
| 129 | 3,5-bis(Trifluormethyl)phenyl | Tetrahydroisoquinolin-1-yl | H |
| 130 | 3,5-bis(Trifluormethyl)phenyl | 2,6-cis-Dimethyl-4-morpholinyl | H |
| 131 | 3,5-bis(Trifluormethyl)phenyl | 4-[(3-Trifluormethyl)phenyl]-1-piperazinyl | H |
| 132 | 3,5-bis(Trifluormethyl)phenyl | 4-*tert*-Butyl-1-piperidinyl | H |
| 133 | 3,5-bis(Trifluormethyl)phenyl | 1-Azepanyl | H |
| 134 | 3,5-bis(Trifluormethyl)phenyl | 4-Benzoyl-1-piperidinyl | H |
| 135 | Phenyl | Tetrahydroisoquinolin-1-yl | H |
| 136 | Phenyl | (4-Methylphenyl)amino | H |
| 137 | 3-Cyanphenyl | 4-(4-Chlorphenyl)-1-piperazinyl | H |
| 138 | 3-Acetylphenyl | Tetrahydroisoquinolin-1-yl | H |
| 139 | 3,5-bis(Trifluormethyl)phenyl | 3-(2-Oxo-1-azepanyl)propylamino | H |
| 140 | 3-Cyanphenyl | N-ethyl-N-phenylamino | H |
| 141 | Phenyl | (4-Chlorphenyl)amino | H |
und pharmazeutisch geeignete Salze davon.

5. Verbindungen nach einem beliebigen der vorangehenden Ansprüche zur Verwendung als Kaliumkanalöffner.

6. Pharmazeutische Zusammensetzung umfassend eine Verbindung nach einem beliebigen der Ansprüche 1 - 4 oder ein pharmazeutisch geeignetes Salz davon mit einer pharmazeutisch geeigneten Säure oder Base, oder einem beliebigen optischen Isomer oder einer Mischung von optischen Isomeren, einschließlich einer racemischen Mischung, oder einer beliebigen tautomeren Form zusammen mit einem oder mehreren pharmazeutisch geeigneten Trägern oder Verdünnungsmitteln.

7. Pharmazeutische Zusammensetzung zur Verwendung in der Behandlung von Krankheiten des endokrinologischen Systems, wie Diabetes, umfassend eine Verbindung nach einem beliebigen der Ansprüche 1 - 4 oder ein pharmazeutisch geeignetes Salz davon mit einer pharmazeutisch geeigneten Säure oder Base oder einem beliebigen optischen Isomer oder einer Mischung von optischen Isomeren, einschließlich einer racemischen Mischung, oder einer beliebigen tautomeren Form zusammen mit einem oder mehreren pharmazeutisch geeigneten Trägern oder Verdünnungsmitteln.

8. Pharmazeutische Zusammensetzung nach Anspruch 6 oder 7 in Form einer oralen Dosierungseinheit oder einer parenteralen Dosierungseinheit.

9. Pharmazeutische Zusammensetzung nach Ansprüchen 6 - 8, wobei die Verbindung in einer Dosis in einem Bereich von ungefähr 0,05 mg bis 1.000 mg verabreicht wird, vorzugsweise von. ungefähr 0,1 mg bis 500 mg und besonders in dem Bereich von 50 mg bis 200 mg pro Tag.

10. Verbindung nach einem beliebigen der Ansprüche 1 - 4 oder ein pharmazeutisch geeignetes Salz davon mit einer pharmazeutisch geeigneten Säure oder Base oder einem beliebigen optischen Isomer oder einer Mischung von optischen Isomeren, einschließlich einer racemischen Mischung, oder einer beliebigen tautomeren Form zur therapeutischen Verwendung.

11. Verbindung nach einem beliebigen der Ansprüche 1 - 4 oder ein pharmazeutisch geeignetes Salz davon mit einer pharmazeutisch geeigneten Säure oder Base oder einem beliebigen optischen Isomer oder einer Mischung von optischen Isomeren, einschließlich einer racemischen Mischung, oder einer beliebigen tautomeren Form zur therapeutischen Verwendung in der Behandlung oder Vorbeugung von Krankheiten des endokrinologischen Systems, wie Diabetes.

12. Verwendung einer Verbindung nach einem beliebigen der Ansprüche 1 - 4 oder eines pharmazeutisch geeigneten Salzes davon mit einer pharmazeutisch geeigneten Säure oder Base oder einem beliebigen optischen Isomer oder einer Mischung von optischen Isomeren, einschließlich einer racemischen Mischung, oder einer beliebigen tautomeren Form zur Zubereitung eines Medikaments.

13. Verwendung einer Verbindung nach einem beliebigen der Ansprüche 1 - 4 oder eines pharmazeutisch geeigneten Salzes davon mit einer pharmazeutisch geeigneten Säure oder Base oder einem beliebigen optischen Isomer oder einer Mischung von optischen Isomeren, einschließlich einer racemischen Mischung, oder einer beliebigen tautomeren Form zur Zubereitung eines Medikaments für die Behandlung oder Vorbeugung von Krankheiten des endokrinologischen Systems, wie Diabetes.

14. Prozess zur Herstellung eines Medikaments, welches zur Behandlung oder Vorbeugung von Krankheiten des endokrinologischen Systems, wie Diabetes, verwendet wird, wobei der Prozess das Bringen einer Verbindung der Formel I nach einem der beliebigen der Ansprüche 1 - 4 oder eines pharmazeutisch geeigneten Salzes davon in eine galenische Dosierungsform umfasst.

15. Verbindung der allgemeinen Formel II 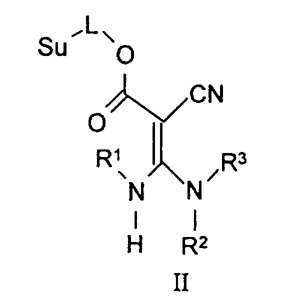 wobei
• Su ein Substrat ist bezugnehmend auf ein beliebiges unlösliches oder teilweise unlösliches Material, an welches Verbindungen kovalent befestigt werden können,
• L eine chemische Bindung oder ein Verknüpfer ("linker") ist, welche/r als ein Molekül mit mindestens zwei reaktiven Stellen definiert ist, welche ihre kovalente Befestigung an andere Moleküle oder an einen Substratverknüpfer erlauben,
• R¹, R² und R³ wie in Anspruch 1 definiert sind.

16. Verbindung nach Anspruch 15, verwendbar zum Screenen nach Kaliumkanalöffnern.

17. Verfahren zur Zubereitung einer Verbindung nach Anspruch 1, umfassend die Schritte:
a) Acylierung eines Substrat-gebundenen Alkohols der Formel Su-L-O-H, wobei Su und L wie in Anspruch 15 definiert sind, mit einem Cyanessigsäurederivat der allgemeinen Struktur NC-CH₂-COX, wobei X eine Hydroxygruppe oder eine Austrittsgruppe ("leaving group") ist;
b) Reaktion des resultierenden Substrat-gebundenen Zwischenproduktes der Formel Su-L-O-CO-CH₂-CN, wobei Su und L wie in Anspruch 15 definiert sind, mit einem aliphatischen oder aromatischen Isothiocyanat der allgemeinen Struktur R¹-NCS, wobei R¹ wie in Anspruch 15 definiert ist, in der Gegenwart einer Base;
c) Reaktion des resultierenden Substrat-gebundenen Zwischenproduktes der Formel Su-L-O-CO-CH(CN)-C(S)(NHR¹) mit einem Amin der allgemeinen Stuktur R²R³NH, wobei R² und R³ wie in Anspruch 1 definiert ist, in Gegenwart eines entschwefelnden Wirkstoffes, um eine Verbindung der Formel II zuzubereiten,
d) Unterwerfung der resultierenden Substrat-gebundenen Verbindung der Formel II an Spaltungsbedingungen, um eine Verbindung der Formel I zuzubereiten.

18. Verfahren nach Anspruch 17, weiterhin umfassend den Schritt des Screenens des Endproduktes der Formel I direkt gegen ein(en) spezischen/s Rezeptor oder Enzym.

19. Verfahren zur Zubereitung einer Verbindung nach Anspruch 15, umfassend die Schritte:
a) Acylierung eines Substrat-gebundenen Alkohols der Formel Su-L-O-H, wobei Su und L wie in Anspruch 15 definiert sind, mit einem Cyanessigsäurederivat der allgemeinen Struktur NC-CH₂-COX, wobei X eine Hydroxygruppe oder eine Austrittsgruppe ist;
b) Reaktion des resultierenden Substrat-gebundenen Zwischenproduktes der Formel Su-L-O-CO-CH₂-CN, wobei Su und L wie in Anspruch 15 definiert sind, mit einem aliphatischen oder aromatischen Isothiocyanat der allgemeinen Struktur R¹-NCS, wobei R¹ wie in Anspruch 15 definiert ist, in der Gegenwart einer Base;
c) Reaktion des resultierenden Substrat-gebundenen Zwischenproduktes der Formel Su-L-O-CO-CH(CN)-C(S)(NHR¹) mit einem Amin der allgemeinen Stuktur R²R³NH, wobei R² und R³ wie in Anspruch 1 definiert ist, in der Gegenwart eines entschwefelnden Reagens, um eine Verbindung der Formel II zuzubereiten.

20. Verfahren nach Anspruch 19, weiterhin umfassend den Schritt des Screenens des Endproduktes der Formel II direkt gegen ein(en) spezifischen/s Rezeptor oder Enzym.

21. Verfahren nach Anspruch 15 bis 20, wobei das entschwefelnde Reagens EDC ist.

22. Verahren nach Anspruch 15 bis 21, wobei die Base für die Reaktion des Isothiocyanats mit dem Cyanessigsäureester ein *tertiäres* Amin ist.

23. Verfahren nach Anspruch 15 bis 22, wobei das Cyanessigsäurederivat das symmetrische Anhydrid ist.

24. Anordnung ("array") umfassend m verschiedene Verbindungen der Formel I an ausgewählten bekannten Positionen in m Behältern, wobei m eine ganze Zahl gleich oder größer als 2 ist.

25. Anordnung umfassend m verschiedene Verbindungen der Formel II, wobei m eine ganze Zahl gleich oder größer als 2 ist, an ausgewählten bekannten Positionen an einem oder mehreren Substraten.

26. Anordnung nach Anspruch 24 oder 25, wobei m zwischen 60 bis 100, vorzugsweise 80, ist.

27. Anordnung umfassend eine Verbindung der Formel I oder n verschiedene Verbindungen der Formel I, wobei n eine ganze Zahl gleich oder größer als 2 ist, an ausgewählten bekannten Positionen in m Behältern, oder an ausgewählten bekannten Positionen an einem Substrat, und eine Verbindung der Formel II oder m-n verschiedene Verbindungen der Formel II, wobei m eine ganze Zahl gleich oder größer als 2 und m>n an ausgewählten bekannten Positionen an einem oder mehreren Substraten ist.

28. Anordnung nach Anspruch 27, wobei m zwischen 60 bis 100, vorzugsweise 80, ist.

29. Anordnung umfassend p verschiedene Mischungen von Verbindungen der Formel I an ausgewählten bekannten Positionen in p Behältern, wobei p eine ganze Zahl gleich oder größer als 2 ist.

30. Anordnung umfassend p verschiedene Mischungen von Verbindungen der Formel II, wobei p eine ganze Zahl gleich oder größer als 2 an ausgewählten bekannten Positionen an einem oder mehreren Substraten ist.

31. Anordnung nach Anspruch 29 oder 30, wobei p zwischen 60 bis 100, vorzugsweise 80, ist.

32. Anordnung umfassend eine Mischung von Verbindungen der Formel I oder r verschiedene Mischungen von Verbindungen der Formel I, wobei r eine ganze Zahl gleich oder größer als 2 an ausgewählten bekannten Positionen in p Behältern oder an ausgewählten bekannten Positionen an einem Substrat ist, und eine Mischung von Verbindungen der Formel II oder p-r verschiedene Mischungen von Verbindungen der Formel II, wobei p eine ganze Zahl gleich oder größer als 2 und p>r an ausgewählten bekannten Positionen an einem oder mehreren Substraten ist.

33. Anordnung nach Anspruch 32, wobei p zwischen 60 bis 100, vorzugsweise 80, ist.

34. Verfahren zur Zubereitung der Anordnung nach Anspruch 25 oder 26 umfassend die Durchführung an ausgewählten bekannten Positionen an einem oder mehreren Substrat(en) der Schritte:
a) gleichzeitige Acylierung eines jeden und aller einzelnen Substrat-gebundenen Alkohole der Formel Su-L-OH, wobei Su und L wie in Anspruch 15 definiert sind, mit einem Cyanessigsäurederivat der allgemeinen Struktur NC-CH₂-COX, wobei X eine Hydroxygruppe oder eine Austrittsgruppe ist;
b) Reaktion eines jeden und aller der resultierenden Substrat-gebundenen Ester der Formel Su-L-O-CO-CH₂-CN, wobei Su und L wie in Anspruch 15 definiert sind, mit einem Isothiocyanat der allgemeinen Struktur R¹-NCS, wobei R¹ wie in Anspruch 15 definiert ist, in der Gegenwart einer Base;
c) Alkylierung eines jeden und aller der resultierenden Substrat-gebundenen Zwischenprodukte der Formel Su-L-O-CO-CH(CN)-C(S)(NHR¹) mit einem Amin der allgemeinen Struktur R²R³NH, wobei R² und R³ wie in Anspruch 1 definiert sind, in der Gegenwart eines entschwefelnden Reagens, um eine Verbindung der Formel II zuzubereiten, befestigt an ein oder mehrere Substrat(e).

35. Verfahren zur Zubereitung der Anordnung nach Anspruch 28 oder 30, wobei das Verfahren nach Anspruch 34 weiterhin den Schritt umfasst:
d) Unterwerfung der resultierenden m Substrat-gebundenen Verbindungen der Formel II an Spaltungsbedingungen, um m Verbindungen der Formel I an ausgewählten bekannten Positionen in m Behältern zuzubereiten, wobei m eine ganze Zahl gleich oder größer als 2 ist.

36. Verfahren zur Zubereitung der Anordnung nach Anspruch 27 oder 28, wobei das Verfahren nach Anspruch 34 weiterhin den Schritt umfasst:
d) Unterwerfung der resultierenden m Substrat-gebundenen Verbindungen der Formel II an Spaltungsbedingungen, um n Verbindungen der Formel I und m-n Verbindungen der Formel II an ausgewählten bekannten Positionen in m Behältern oder an ausgewählten bekannten Positionen an einem Substrat zuzubereiten.

37. Verfahren nach einem beliebigen der Ansprüche 34, 35 oder 36, wobei die Base für die Reaktion des Isothiocyanats mit der Cyanessigsäure in Anspruch 34, Schritt b) ein *tertiäres* Amin ist.

38. Verfahren nach einem beliebigen der Ansprüche 34 bis 37, wobei das Cyanessigsäurederivat das symmetrische Anhydrid ist.

39. Verfahren nach einem beliebigen der Ansprüche 34 bis 38, weiterhin umfassend das Screenen der Endprodukte direkt gegen ein(en) spezifischen/s Rezeptor oder Enzym.

40. Anordnung von Verbindungen der Formel I nach Anspruch 24 oder 26, wobei R¹ Phenyl, 4-Trifluormethylphenyl, 2-Trifluormethylphenyl, 2,3-Dichlorphenyl, 3-Chlor-4-fluorphenyl, 2-Methoxy-4-nitrophenyl, 2,4-Difluorphenyl, 4-Cyanphenyl, 3,5-bis(Trifluormethyl)phenyl, 3-Fluorphenyl, 3-Pyridyl, 3,4-Dichlorphenyl oder Benzyl ist.

41. Anordnung von Verbindungen der Formel I nach einem beliebigen der Ansprüche 24, 26 oder 39, wobei R² 2-Methylpropyl, 1,2-Dimethylpropyl, Isopropyl, 1,3-Dimethylbutyl, 2,2-Dimethylpropyl, Butyl, 4-*tert*-Butylcyclohexyl, 1,2,2-trimethylpropyl, exo-2-Norbornyl und Cyclohexylmethyl, 4-Methoxybenzyl, Phenyl, Propyl, Hexyl, Propargyl oder 3-Methylbutyl ist; oder
R² und R³ durch -(CH₂)ₙ-, wobei n 4-7 ist, miteinander verknüpft sind.

42. Verwendung einer Anordnung nach einem beliebigen der Ansprüche 24 bis 33 zum Screenen nach Verbindungen der Formel I gegen spezifische Rezeptoren oder Enzyme.

43. Verwendung einer Anordnung nach einem beliebigen der Ansprüche 24 bis 33 zum Screenen von Verbindungen der Formel I gegen Kaliumkanäle.

## Revendications

1. Un composé de formule générale I 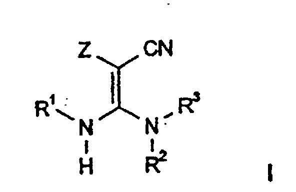 dans laquelle
R¹ est un radical aryle éventuellement substitué ;
R² et R³ sont indépendamment de l'hydrogène, un radical alkyle éventuellement substitué avec un radical aryle, hétéroaryle, un système hétérocyclique à 5, 6 ou 7 chaînons, de l'halogène, un radical hydroxy, alcoxy, aryloxy, alkylthio, arylthio, dialkylamino, arylalkylamino ou diarylamino, un radical aryle éventuellement substitué avec un radical alkyle, aryle, hétéroaryle, de l'halogène, un radical alcoxy, aryloxy, dialkylamino, alkylarylamino, diarylamino, nitro, alkylsulfonyle, aryl-sulfonyle, cyano, alcoxycarbonyle ou aminocarbonyle, un radical hétéroaryle éventuellement substitué avec un radical alkyle, aryle, hétéroaryle, de l'halogène, un radical alcoxy, aryloxy, dialkylamino, alkylarylamino, diarylamino, de l'halogène, un radical nitro, alkyl-sulfonyle, aryl-sulfonyle, cyano, alcoxycarbonyle ou aminocarbonyle ; ou
R² et R³ sont reliés conjointement par -(CH₂)ₙ-, n étant 4 à 7, à condition que R² et R³ ne soient pas en même temps de l'hydrogène ;
Z est de l'hydrogène ; et
ses sels acceptables du point de vue pharmaceutique.

2. Un composé selon la revendication 1, dans lequel R¹ est éventuellement un radical phényle substitué.

3. Un composé selon la revendication 2, dans lequel R¹ est un radical phényle substitué par un ou deux halogènes, groupes perhalométhyle ou cyano.

4. Un composé qui est choisi parmi le groupe comprenant : 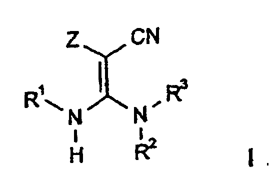
| No. | R¹ | -NR²R³ | Z |
|---|---|---|---|
| 1 | 4-(trifluorométhyl)phényle | 2-(méthylpropyl)amino | H |
| 2 | 4-(trifluorométhyl)phényle | (1,2-diméthylpropyl)amino | H |
| 3 | 4-(trifluorométhyl)phényle | isopropylamino | H |
| 4 | 4-(trifluorométhyl)phényle | (1,3-diméthylbutyl)amino | H |
| 5 | 4-(trifluorométhyl)phényle | (2,2-diméthylpropyl)amino | H |
| 6 | 4-(trifluorométhyl)phényle | butylamino | H |
| 7 | 4-(trifluorométhyl)phényle | (4-tert-butylcyclohexyl)amino | H |
| 8 | 4-(trifluorométhyl)phényle | (1,2,2-triméthylpropyl)amino | H |
| 9 | 4-(trifluorométhyl)phényle | 2-exo-norbornylamino | H |
| 10 | 4-(trifluorométhyl)phényle | (cyclohexylméthyl)amino | H |
| 11 | 2-(trifluorométhyl)phényle | 2-(méthylpropyl)amino | H |
| 12 | 2-(trifluorométhyl)phényle | (1,2-diméthylpropyl)amino | H |
| 13 | 2-(trifluorométhyl)phényle | isopropylamino | H |
| 14 | 2-(trifluorométhyl)phényle | (1,3-diméthylbutyl)amino | H |
| 15 | 2-(trifluorométhyl)phényle | (2,2-diméthylpropyl)amino | H |
| 16 | 2-(trifluorométhyl)phényle | butylamino | H |
| 17 | 2-(trifluorométhyl)phényle | (4-tert-butylcyclohexyl)amino | H |
| 18 | 2-(trifluorométhyl)phényle | (1,2,2-triméthylpropyl)amino | H |
| 19 | 2-(trifluorométhyl)phényle | 2-exo-norbornylamino | H |
| 20 | 2-(trifluorométhyl)phényle | (cyclohexylméthyl)amino | H |
| 21 | 2,3-dichlorophényle | 2-(méthylpropyl)amino | H |
| 22 | 2,3-dichlorophényle | (1,2-diméthylpropyl)amino | H |
| 23 | 2,3-dichlorophényle | isopropylamino | H |
| 24 | 2,3-dichlorophényle | (1,3-diméthylbutyl)amino | H |
| 25 | 2,3-dichlorophényle | 2,2-diméthylpropyl)amino | H |
| 26 | 2,3-dichlorophényle | butylamino | H |
| 27 | 2,3-dichlorophényle | (4-tert-butylcyclohexyl)amino | H |
| 28 | 2,3-dichlorophényle | (1,2,2-triméthylpropyl)amino | H |
| 29 | 2,3-dichlorophényle | 2-exo-norbornylamino | H |
| 30 | 2,3-dichlorophényle | (cyclohexylméthyl)amino | H |
| 31 | 2-méthoxy-4-nitrophényle | 2-(méthylpropyl)amino | H |
| 32 | 2-méthoxy-4-nitrophényle | (1,2-diméthylpropyl)amino | H |
| 33 | 2-méthoxy-4-nitrophényle | isopropylamino | H |
| 34 | 2-méthoxy-4-nitrophényle | (1,3-diméthylbutyl)amino | H |
| 35 | 2-méthoxy-4-nitrophényle | (2,2-diméthylpropyl)amino | H |
| 36 | 2-méthoxy-4-nitrophényle | butylamino | H |
| 37 | 2-méthoxy-4-nitrophényle | (4-tert-butylcyclohexyl)amino | H |
| 38 | 2-méthoxy-4-nitrophényle | (1,2,2-triméthylpropyl)amino | H |
| 39 | 2-méthoxy-4-nitrophényle | 2-exo-norbornylamino | H |
| 40 | 2-méthoxy-4-nitrophényle | (cyclohexylméthyl)amino | H |
| 41 | 2,4-difluorophényle | 2-(méthylpropyl)amino | H |
| 42 | 2,4-difluorophényle | (1,2-diméthylpropyl)amino | H |
| 43 | 2,4-difluorophényle | isopropylamino | H |
| 44 | 2,4-difluorophényle | (1,3-diméthylbutyl)amino | H |
| 45 | 2,4-difluorophényle | (2,2-diméthylpropyl)amino | H |
| 46 | 2,4-difluorophényle | butylamino | H |
| 47 | 2,4-difluorophényle | (4-tert-butylcyclohexyl)amino | H |
| 48 | 2,4-difluorophényle | (1,2,2-triméthylpropyl)amino | H |
| 49 | 2,4-difluorophényle | 2-exo-norbornylamino | H |
| 50 | 2,4-difluorophényle | (cyclohexylméthyl)amino | H |
| 51 | 3-chloro-4-fluorophényle | 2-(méthylpropyl)amino | H |
| 52 | 3-chloro-4-fluorophényle | (1,2-diméthylpropyl)amino | H |
| 53 | 3-chloro-4-fluorophényle | isopropylamino | H |
| 54 | 3-chloro-4-fluorophényle | (1,3-diméthylbutyl)amino | H |
| 55 | 3-chloro-4-fluorophényle | (2,2-diméthylpropyl)amino | H |
| 56 | 3-chloro-4-fluorophényle | butylamino | H |
| 57 | 3-chloro-4-fluorophényle | (4-tert-butylcyclohexyl)amino | H |
| 58 | 3-chloro-4-fluorophényle | (1,2,2-triméthylpropyl)amino | H |
| 59 | 3-chloro-4-fluorophényle | 2-exo-norbornylamino | H |
| 60 | 3-chloro-4-fluorophényle | (cyclohexylméthyl)amino | H |
| 61 | 4-cyanophényle | 2-(methylpropyl)amino | H |
| 62 | 4-cyanophényle | (1,2-diméthylpropyl)amino | H |
| 63 | 4-cyanophényle | isopropylamino | H |
| 64 | 4-cyanophényle | (1,3-diméthylbutyl)amino | H |
| 65 | 4-cyanophényle | (2,2-diméthylpropyl)amino | H |
| 66 | 4-cyanophényle | butylamino | H |
| 67 | 4-cyanophényle | (4-tert-butylcyclohexyl)amino | H |
| 68 | 4-cyanophényle | (1,2,2-triméthylpropyl)amino | H |
| 69 | 4-cyanophényle | 2-exo-norbornylamino | H |
| 70 | 4-cyanophényle | (cyclohexylméthyl)amino | H |
| 71 | 3,5-bis(trifluorométhyl)phényle | 2-(méthylpropyl)amino | H |
| 72 | 3,5-bis(trifluorométhyl)phényle | (1,2-diméthylpropyl)amino | H |
| 73 | 3,5-bis(trifluorométhyl)phényle | isopropylamino | H |
| 74 | 3,5-bis(trifluorométhyl)phényle | (1,3-diméthylbutyl)amino | H |
| 75 | 3,5-bis(trifluorométhyl)phényle | (2,2-diméthylpropyl)amino | H |
| 76 | 3,5-bis(trifluorométhyl)phényle | butylamino | H |
| 77 | 3,5-bis (trifluorométhyl)phényle | (4-tert-butylcyclohexyl)amino | H |
| 78 | 3,5-bis(trifluorométhyl)phényle | (1,2,2-triméthylpropyl)amino | H |
| 79 | 3,5-bis(trifluorométhyl)phényle | 2-exo-norbornylamino | H |
| 80 | 3,5-bis(trifluorométhyl)phényle | (cyclohexylméthyl)amino | H |
| 81 | phényle | (4-méthoxybenzyl)amino | H |
| 82 | 3-fluorophényle | propylamino | H |
| 83 | 3-fluorophényle | hexylamino | H |
| 84 | 3-fluorophényle | propargylamino | H |
| 85 | 3-fluorophényle | (3-méthylbutyl)amino | H |
| 86 | 3-pyridyle | propylamino | H |
| 87 | 3-pyridyle | hexylamino | H |
| 88 | 3-pyridyle | propargylamino | H |
| 89 | 3-pyridyle | (3-méthylbutyl)amino | H |
| 90 | 3,4-dichlorophényle | propylamino | H |
| 91 | 3,4-dichlorophényle | hexylamino | H |
| 92 | 3,4-dichlorophényle | propargylamino | H |
| 93 | 3,4-dichlorophényle | (3-méthylbutyl)amino | H |
| 94 | 3,5-bis(trifluorométhyl)phényle | propylamino | H |
| 95 | 3,5-bis(trifluorométhyl)phényle | hexylamino | H |
| 96 | 3,5-bis(trifluorométhyl)phényle | propargylamino | H |
| 97 | 3,5-bis(trifluorométhyl)phényle | (3-méthylbutyl)amino | H |
| 98 | phényle | phénylamino | H |
| 99 | benzyle | (4-méthoxybenzyl)amino | H |
| 100 | phényle | 1-pyrrolidinyle | H |
| 101 | 3-(trifluorométhyl)phényle | (3-méthylbutyl) amino | H |
| 102 | 4-chloro-3-(trifluorométhyl)phényle | (3-méthylbutyl)amino | H |
| 103 | 3-acétylphényle | (3-méthylbutyl)amino | H |
| 104 | 2-chloro-5-(trifluorométhyl)phényle | (3-méthylbutyl)amino | H |
| 105 | 3,4-dicyanophényle | (3-méthylbutyl)amino | H |
| 106 | 4-bromo-2-(trifluorométhyl)phényle | (3-méthylbutyl)amino | H |
| 107 | 4,6-diméthyl-2-pyrimidinyle | (3-méthylbutyl)amino | H |
| 108 | 4-acétylphényle | (3-méthylbutyl)amino | H |
| 109 | 3,5-dichlorophényle | (3-méthylbutyl)amino | H |
| 110 | 3-chloro-4-méthylphényle | (3-méthylbutyl)amino | H |
| 111 | 2,5-bis(trifluorométhyl)phényle | (3-méthylbutyl)amino | H |
| 112 | 3,5-dichlorophényle | (2-méthylpropyl)amino | H |
| 113 | 3-chloro-4-méthylphényle | (2-méthylpropyl)amino | H |
| 114 | 2,5-bis(trifluorométhyl)phényle | (2-méthylpropyl)amino | H |
| 115 | 3,5-bis(trifluorométhyl)phényle | (3-phénylpropyl)amino | H |
| 116 | 3,5-bis(trifluorométhyl)phényle | (2,2,6,6-tétraméthyl-4-pipéridinyl)amino | H |
| 117 | 3,5-bis(trifluorométhyl)phényle | N,N-dipropylamino | H |
| 118 | 3,5-bis(trifluorométhyl)phényle | 2-(4-chlorophényl)éthylamino | H |
| 119 | 3,5-bis(trifluorométhyl)phényle | 2-(2-pyridyl)éthylamino | H |
| 120 | 3,5-bis(trifluorométhyl)phényle | 4-méthyl-1-pipéridyle | H |
| 121 | 3,5-bis(trifluorométhyl)phényle | N,N-bis(2-méthylpropyl)amino | H |
| 122 | 3,5-bis(trifluorométhyl)phényle | 1-pyrrolidinyle | H |
| 123 | 3,5-bis(trifluorométhyl)phényle | 3-(1-imidazolyl)propylamino | H |
| 124 | 3,5-bis(trifluorométhyl)phényle | N-méthyl-N-(3-pyridyl) méthylamino | H |
| 125 | 3,5-bis(trifluorométhyl)phényle | (3-amine-2,2-diméthylpropyl) amino | H |
| 126 | 3,5-bis(trifluorométhyl)phényle | 3-(2-oxo-1-pyrrolidinyl) propylamino | H |
| 127 | 3,5-bis(trifluorométhyl)phényle | (4-méthoxybenzyl)amino | H |
| 128 | 3,5-bis(trifluorométhyl)phényle | 3-hydroxy-1-pipéridinyle | H |
| 129 | 3,5-bis(trifluorométhyl)phényle | tétrahydroisoquinolin-1-yle | H |
| 130 | 3,5-bis(trifluorométhyl)phényle | 2,6-cis-diméthyl-4-morpholinyle | H |
| 131 | 3,5-bis(trifluorométhyl)phényle | 4-[(3-trifluorométhyl)phényl]-1-pipérazinyle | H |
| 132 | 3,5-bis(trifluorométhyl)phényle | 4-tert-butyl-1-pipéridinyle | H |
| 133 | 3,5-bis(trifluorométhyl)phényle | 1-azépanyle | H |
| 134 | 3,5-bis(trifluorométhyl)phényle | 4-benzoyl-1-pipéridinyle | H |
| 135 | phényle | tétrahydroisoquinolin-1-yle H | |
| 136 | phényle | (4-méthylphényl)amino | H |
| 137 | 3-cyanophényle | 4-(4-chlorophényl)-1-pipérazinyle | H |
| 138 | 3-acétylphényle | tétrahydroisoquinolin-1-yle | H |
| 139 | 3,5-bis(trifluorométhyl)phényle | 3-(2-oxo-1-azépanyl)propylamino | H |
| 140 | 3-cyanophényle | N-éthyl-N-phénylamino | H |
| 141 | phényle | (4-chlorophényl)amino | H |
et ses sels acceptables du point de vue pharmaceutique.

5. Composés selon l'une quelconque des revendications précédentes, destinés à être utilisés comme agents d'ouverture de canal au potassium.

6. Une composition pharmaceutique comprenant un composé selon l'une quelconque des revendications 1 à 4 ou un sel acceptable du point de vue pharmaceutique de celui-ci avec un acide ou une base acceptable du point de vue pharmaceutique ou n'importe quel isomère optique ou mélange d'isomères optiques, y compris un mélange racémique ou n'importe quelle forme tautomère avec un ou plusieurs véhicules ou diluants acceptables du point de vue pharmaceutique.

7. Une composition pharmaceutique destinée à être utilisée dans le traitement de maladies du système endocrinien comme le diabète, comprenant un composé selon l'une quelconque des revendications 1 à 4 ou un sel acceptable du point de vue pharmaceutique de celui-ci avec un acide ou une base acceptable du point de vue pharmaceutique ou n'importe quel isomère optique ou mélange d'isomères optiques, y compris un mélange racémique ou n'importe quelle forme tautomère conjointement avec un ou plusieurs véhicules ou diluants acceptables du point de vue pharmaceutique.

8. La composition pharmaceutique selon la revendication 6 ou 7 sous la forme d'une unité à dosage oral ou d'une unité à dosage parentéral.

9. Une composition pharmaceutique selon l'une des revendications 6 à 8, dans laquelle ledit composé est administré selon une dose dans la gamme d'environ 0,05 mg à 1 000 mg, de préférence d'environ 0,1 mg à 500 mg et, notamment, dans la gamme de 50 mg à 200 mg par jour.

10. Un composé selon l'une quelconque des revendications 1 à 4 ou un sel acceptable du point de vue pharmaceutique de celui-ci avec un acide ou une base acceptable du point de vue pharmaceutique, ou n'importe quel isomère optique ou mélange d'isomères optiques, y compris un mélange racémique ou n'importe quelle forme tautomère pour une utilisation thérapeutique.

11. Un composé selon l'une quelconque des revendications 1 à 4 ou un sel acceptable du point de vue pharmaceutique de celui-ci avec un acide ou une base acceptable du point de vue pharmaceutique, ou n'importe quel isomère optique ou mélange d'isomères optiques, y compris un mélange racémique ou n'importe quelle forme tautomère pour une utilisation thérapeutique dans le traitement ou la prévention de maladies du système endocrinien comme le diabète.

12. Utilisation d'un composé selon l'une quelconque des revendications 1 à 4 ou un sel acceptable du point de vue pharmaceutique de celui-ci avec un acide ou une base acceptable du point de vue pharmaceutique, ou n'importe quel isomère optique ou mélange d'isomères optiques, y compris un mélange racémique ou n'importe quelle forme tautomère pour préparer un médicament.

13. Utilisation d'un composé selon l'une quelconque des revendications 1 à 4 ou d'un sel acceptable du point de vue pharmaceutique de celui-ci avec un acide ou une base acceptable du point de vue pharmaceutique, ou n'importe quel isomère optique ou mélange d'isomères optiques, y compris un mélange racémique ou n'importe quelle forme tautomère pour la préparation d'un médicament pour le traitement ou la prévention de maladies du système endocrinien comme le diabète.

14. Un procédé pour la fabrication d'un médicament destiné à être utilisé dans le traitement ou la prévention de maladies du système endocrinien comme le diabète, lequel procédé consiste à amener un composé de formule I selon l'une quelconque des revendications 1 à 4 ou un sel acceptable du point de vue pharmaceutique de celui-ci selon une forme de dosage galénique.

15. Un composé de formule générale II 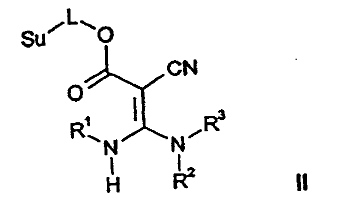 dans laquelle
• Su est un substrat se référant à n'importe quel matériau insoluble ou partiellement insoluble auquel les composés peuvent être fixés de façon covalente,
• L est une liaison chimique ou un agent de liaison qui est défini comme une molécule avec au moins deux sites réactifs, qui permettent sa fixation par covalence à d'autres molécules ou à un dispositif de liaison de substrat,
• R¹, R² et R³ sont comme défini dans la revendication 1.

16. Un composé selon la revendication 15 utile pour cribler les agents d'ouverture de canaux au potassium.

17. Un procédé pour préparer un composé selon la revendication 1, comprenant les étapes de :
a) acylation d'un alcool lié au substrat de formule Su-L-O-H, dans laquelle Su et L sont comme défini dans la revendication 15, avec un dérivé d'acide cyanoacétique de structure générale NC-CH₂-COX, dans laquelle X est un groupe hydroxy ou un groupe labile ;
b) réaction de l'intermédiaire -lié au substrat résultant de la formule Su-L-O-CO-CH₂-CN dans laquelle Su et L sont comme défini dans la revendication 15, avec un isothiocyanate aliphatique ou aromatique de structure générale R¹-NCS dans laquelle R¹ est comme défini dans la revendication 15 en présence d'une base ;
c) réaction de l'intermédiaire lié au substrat résultant de la formule Su-L-O-CO-CH(CN)-C(S)(NHR¹) avec une amine de structure générale R²R³NH, dans laquelle R² et R³ sont comme défini dans la revendication 1, en présence d'un agent désulfurant afin de préparer un composé de formule II ;
d) soumission du composé lié au substrat résultant de formule II à des conditions de clivage afin de préparer un composé de formule I.

18. Le procédé selon la revendication 17 comprenant, en outre, l'étape de criblage du produit final de formule I directement contre un récepteur ou enzyme spécifique.

19. Un procédé de préparation d'un composé selon la revendication 15, comprenant les étapes de :
a) acylation d'un alcool lié au substrat de formule Su-L-O-H, dans laquelle Su et L sont comme défini dans la revendication 15, avec un dérivé d'acide cyanoacétique de structure générale NC-CH₂-COX, dans laquelle X est un groupe hydroxy ou un groupe labile ;
b) réaction de l'intermédiaire lié au substrat résultant de la formule Su-L-O-CO-CH₂-CN dans laquelle Su et L sont comme défini dans la revendication 15, avec un isothiocyanate aliphatique ou aromatique de structure générale R¹-NCS dans laquelle R¹ est comme défini dans la revendication 15 en présence d'une base ;
c) réaction de l'intermédiaire lié au substrat résultant de la formule Su-L-O-CO-CH(CN)-C(S)(NHR¹) avec une amine de structure générale R²R³NH, dans laquelle R² et R¹ sont comme défini dans la revendication 1, en présence d'un réactif désulfurant afin de préparer un composé de formule II.

20. Le procédé selon la revendication 19 comprenant, en outre, l'étape de criblage du produit final de formule II directement contre un récepteur ou enzyme spécifique.

21. Le procédé selon l'une des revendications 15 à 20, dans lequel le réactif de désulfuration est EDC.

22. Le procédé selon l'une des revendications 15 à 21, dans lequel la base pour la réaction de l'isothiocyanate avec l'ester d'acide cyanoacétique est une aminé tertiaire.

23. Le procédé selon l'une des revendications 15 à 22, dans lequel le dérivé d'acide cyanoacétique est l'anhydride symétrique.

24. Un réseau comprenant m composés différents de formule I, dans des positions connues choisies dans m conteneurs, où m est un entier égal ou supérieur à 2.

25. Un réseau comprenant m composés différents de formule II, où m est un entier égal ou supérieur à 2, dans des positions connues choisies sur un ou plusieurs substrats.

26. Le réseau selon la revendication 24 ou 25, dans lequel m est compris entre 60 et 100 et est de préférence 80.

27. Un réseau comprenant un composé de formule I ou n composés différents de formule I, dans lequel n est un entier égal ou supérieur à 2, dans des positions connues choisies dans m conteneurs ou dans des positions connues choisies sur un substrat et un composé de formule II ou m-n composés différents de formule II où m est un entier égal ou supérieur à 2 et m > n aux positions connues choisies sur un ou plusieurs substrats.

28. Le réseau selon la revendication 27, dans lequel m est compris entre 60 et 100 et est de préférence 80.

29. Un réseau comprenant p mélanges différents de composés de formule I, aux positions connues choisies dans p conteneurs, où p est un entier égal ou supérieur à 2.

30. Un réseau comprenant p mélanges différents de composés de formule II, où p est un entier égal ou supérieur à 2 dans des positions connues choisies d'un ou plusieurs substrats.

31. Le réseau selon la revendication 29 ou 30, dans lequel p est compris entre 60 et 100 et est de préférence 80.

32. Un réseau comprenant un mélange de composés de formule I ou r mélanges différents de composés de formule I, où r est un entier égal ou supérieur à 2, dans des positions connues choisies dans p conteneurs ou aux positions connues choisies sur un substrat et un mélange de composés de formule II ou p-r mélanges différents de composés de formule II où p est un entier égal ou supérieur à 2 et p > r aux positions connues choisies sur un ou plusieurs substrats.

33. Le réseau selon la revendication 32, dans lequel p est compris entre 60 et 100 et est de préférence 80.

34. Un procédé pour préparer le réseau selon la revendication 25 ou 26, comprenant la mise en oeuvre dans des positions connues choisies sur un ou plusieurs substrat(s) des étapes de :
a) acylation simultanée de chacun et chaque alcool lié au substrat seul de formule Su-L-OH, où Su et L sont comme défini dans la revendication 15, avec un dérivé d'acide cyanoacétique de structure générale NC-CH₂-COX, dans laquelle X est un groupe hydroxy ou un groupe labile ;
b) réaction de chacun et chaque ester lié au substrat résultant de la formule Su-L-O-CO-CH₂-CN dans laquelle Su et L sont comme défini dans la revendication 15, avec un isothiocyanate de structure générale R¹-NCS dans laquelle R¹ est comme défini dans la revendication 15 en présence d'une base ;
c) alkylation de chacun et chaque intermédiaire lié au substrat résultant de la formule Su-L-O-CO-CH(CN)-C(S)(NHR¹) avec une amine de structure générale R²R³NH, dans laquelle R² et R³ sont comme défini dans la revendication 1, en présence d'un agent désulfurant afin de préparer un composé de formule II fixé à un ou plusieurs substrat(s).

35. Un procédé pour préparer le réseau selon la revendication 28 ou 30, selon le procédé de la revendication 34, comprenant, en outre, l'étape de :
d) soumission des m composés liés au substrat résultant de formule II à des conditions de clivage afin de préparer m composés de formule I, aux positions connues choisies dans m conteneurs où m est un entier égal ou supérieur à 2.

36. Un procédé pour préparer le réseau selon la revendication 27 ou 28, selon le procédé de la revendication 34, comprenant, en outre, l'étape de :
d) soumission des m composés liés au substrat résultant de formule II à des conditions de clivage afin de préparer n composés de formule I, et m-n composés de formule II aux positions connues choisies dans m conteneurs ou aux positions connues choisies sur un substrat.

37. Le procédé selon l'une quelconque des revendications 34, 35 ou 36 dans lequel la base pour la réaction de l'isothiocyanate avec l'acide cyanoacétique dans la revendication 34, étape b) est une aminé tertiaire.

38. Le procédé selon l'une quelconque des revendications 34 à 37, dans lequel le dérivé d'acide cyanoacétique est l'anhydride symétrique.

39. Le procédé selon l'une quelconque des revendications 34 à 38 comprenant, en outre, le criblage des produits finaux directement contre un récepteur ou enzyme spécifique.

40. Le réseau de composés de formule I, selon la revendication 24 ou 26, dans lequel R¹ est un radical phényle, 4-trifluorométhylphényle, 2-trifluorométhylphényle, 2,3-dichlorophényle, 3-chloro-4-fluorophényle, 2-méthoxy-4-nitrophényle, 2,4-difluorophényle, 4-cyanophényle, 3,5-bis (trifluorométhyl)phényle, 3-fluorophényle, 3-pyridyle, 3,4-dichlorophényle ou benzyle.

41. Le réseau de composés de formule I, selon l'une quelconque des revendications 24, 26 ou 39, dans lequel R² est le radical 2-méthylpropyle, 1,2-diméthylpropyle, isopropyle, 1,3-diméthylbutyle, 2,2-diméthylpropyle, butyle, 4-tert-butylcyclohexyle, 1,2,2-triméthylpropyle, exo-2-norbornyle et cyclohexylméthyle, 4-méthoxybenzyle, phényle, propyle, hexyle, propargyle ou 3-méthylbutyle ; ou
R² et R³ sont reliés conjointement par -(CH₂)ₙ-, n étant 4-7.

42. Utilisation d'un réseau selon l'une quelconque des revendications 24 à 33 pour cribler des composés de formule I contre des enzymes ou récepteurs spécifiques.

43. Utilisation d'un réseau selon l'une quelconque des revendications 24 à 33 pour cribler des composés de formule I contre des canaux au potassium.
